# EUROPEAN PATENT SPECIFICATION

(11) **EP 3 200 585 B1**
(45) Date of publication and mention of the grant of the patent: **11.03.2020**
(21) Application number: 15794317.6
(22) Date of filing: 01.10.2015
(51) Int. Cl.: A01N 25/26, A61K 31/717, A61K 31/765

(54) **ANTIFOULING METHOD USING LIPOSOME AND POLYMER-CONTAINING COMPOSITIONS**
VERFAHREN ZUR FÄULNISVERHINDERUNG MIT LIPOSOM- UND POLYMERHALTIGEN ZUSAMMENSETZUNGEN
PROCÉDÉ ANTI-SALISSURES À L'AIDE DE LIPOSOME ET COMPOSITIONS CONTENANT UN POLYMÈRE ET DES LIPOSOMES

(30) Priority: 01.10.2014 IL 23492914
(43) Date of publication of application: 09.08.2017
(73) Proprietor: Yeda Research and Development Co., Ltd., 7610002 Rehovot (IL)
(72) Inventor: KLEIN, Jacob, 7634501 Rehovot (IL); GOLDBERG, Ronit, 7610002 Rehovot (IL); LIN, Weifeng, 7610002 Rehovot (IL); JAHN, Sabrina, 7610002 Rehovot (IL)
(74) Representative: Dennemeyer & Associates S.A.
(86) International application number: PCT/IL2015/050987
(87) International publication number: WO 2016/051413

(56) References cited:
- EP-A1- 0 319 638
- US-A- 4 348 329
- US-A- 6 103 246
- US-A- 6 132 765
- US-A1- 2009 039 035
- US-A1- 2010 239 651
- US-A1- 2011 052 656
- US-B2- 7 824 557
- PETER KINGSHOTT ET AL: "Surfaces that resist bioadhesion", CURRENT OPINION IN SOLID STATE AND MATERIALS SCIENCE, vol. 4, no. 4, 1 August 1999 (1999-08-01), pages 403-412, XP055232738, GB ISSN: 1359-0286, DOI: 10.1016/S1359-0286(99)00018-2
- ANNE DANION ET AL: "Antibacterial activity of contact lenses bearing surface-immobilized layers of intact liposomes loaded with levofloxacin", JOURNAL OF PHARMACEUTICAL SCIENCES, vol. 96, no. 9, 1 January 2007 (2007-01-01), pages 2350-2363, XP055232750, WASHINGTON, US ISSN: 0022-3549, DOI: 10.1002/jps.20871
- BUDAI ET AL: "Gels and liposomes in optimized ocular drug delivery: Studies on ciprofloxacin formulations", INTERNATIONAL JOURNAL OF PHARMACEUTICS, ELSEVIER BV, NL, vol. 343, no. 1-2, 30 August 2007 (2007-08-30), pages 34-40, XP022223249, ISSN: 0378-5173, DOI: 10.1016/J.IJPHARM.2007.04.013
- XINMING L ET AL: "Polymeric hydrogels for novel contact lens-based ophthalmic drug delivery systems: A review", CONTACT LENS AND ANTERIOR EYE, STOCKTON PRESS, BASINGSTOKE, GB, vol. 31, no. 2, 1 April 2008 (2008-04-01), pages 57-64, XP022613260, ISSN: 1367-0484, DOI: 10.1016/J.CLAE.2007.09.002 [retrieved on 2007-10-25]
- HU, X.; HAO, L.; WANG, H. ET AL.: "Hydrogel Contact lens for extended delivery of ophthalmic drugs", INTERNATIONAL JOURNAL OF POLYMER SCIENCES, vol. 2011, 2011, pages 1-9, XP009187480,
- VERBERNE G ET AL: "Liposomes as potential biolubricant additives for wear reduction in human synovial joints", WEAR, ELSEVIER SEQUOIA, LAUSANNE, CH, vol. 268, no. 7-8, 9 March 2010 (2010-03-09), pages 1037-1042, XP026924037, ISSN: 0043-1648, DOI: 10.1016/J.WEAR.2009.12.037 [retrieved on 2010-02-24]
- XU J ET AL: "Cyclodextrin-containing hydrogels for contact lenses as a platform for drug incorporation and release", ACTA BIOMATERIALIA, ELSEVIER, AMSTERDAM, NL, vol. 6, no. 2, 1 February 2010 (2010-02-01), pages 486-493, XP026811140, ISSN: 1742-7061 [retrieved on 2009-07-18]
- DANION, A.; BROCHU, H. ET AL.: "Fabrication and characterization of contact lenses bearing surface-immobilized layers of intact liposomes", J BIOMED MATER RES A, 30 January 2007 (2007-01-30), pages 41-51, XP9187491,

## Description

The present disclosure, in some aspects thereof, relates to material science and, more particularly, but not exclusively, to compositions and methods for reducing biofilm formation on a surface of various substrates.

Fouling of surfaces by adsorption of unwanted material from fluids with which they are in contact can lead to highly undesirable effects. The fouling material may consist of a non-living substance or living organisms. Fouling by microorganisms, such as bacteria, may pose a health threat.

A biofilm is a group of microorganisms, such as bacteria or fungi, in which cells stick to each other on a surface. The adherent cells are frequently embedded within a self-produced matrix composed of extracellular DNA, proteins and polysaccharides. Microbial cells in a biofilm are physiologically distinct from free, individual cells in a liquid medium, which are referred to as "planktonic" cells.

Biofilm formation on a substrate may be facilitated by a "conditioning film", formed by adsorption of various substances, such as proteins and polysaccharides, onto the substrate surface. Microorganisms may then be capable of adhering to the substrate to a far greater extent than in the absence of the conditioning film.

In addition to posing potential health threats, biofilms may also promote corrosion. By acting as a diffusion barrier which reduces local concentrations of oxygen, biofilms can accelerate the corrosion of a metallic substrate by creating a differential aeration concentration cell. The corrosion and weathering caused by biofilm can lead to considerable damage to heat exchangers, unexpected corrosion of stainless steel, and premature destruction of membranes, and many other technological, industrial and homestead aliments.

Infections associated with medical devices in contact with human tissue (especially implanted devices such as stents or catheters) typically begin with the adhesion of bacteria to a surface, and their subsequent proliferation, resulting in a biofilm, in which the bacteria become highly antibiotic-resistant. For example, neutralizing bacteria in a biofilm may require a 1000-fold dose of antibiotic compared to planktonic bacteria [Kostina et al., Biomacromolecules 2012, 13:4164-4170]. Such infections are frequent - approximately 4 % of all implanted vascular grafts and medical heart valves, 2 % of implanted joint prostheses and 5 % of fracture fixation devices become infected [Kostina et al., Biomacromolecules 2012, 13:4164-4170]. The cost of curing such infections may exceed 50,000$ per case, apart from suffering or morbidity. Catheters in particular are often colonized by bacteria, with resultant infection, suffering and costs. Such infections represent about half of all hospital associated infections (HAI), and are especially costly and difficult to treat.

Efforts have been made to develop materials which exhibit reduced protein adhesion, with the goal of reducing adhesion of microbes that form biofilms, as microbial adhesion is mediated by surface proteins.

Hydrogels formed by copolymerization of carboxybetaine monomers with 2-hydroxyethyl methacrylate (HEMA) have been reported to exhibit low levels of fouling from blood plasma [Kostina et al., Biomacromolecules 2012, 13:4164-4170]. Modification of polysulfone (PSf) membranes with 2-methacryloxyethyl phosphorylcholine (MPC) polymer was reported to decrease protein adsorption [Ishihara et al., Biomaterials 1999, 20:1553-1559]. Other studies have associated zwitterionic hydrogel surfaces with enhanced antifouling properties [Schlenoff, Langmuir 2014, 30:9625-9636; Yang et al., J Mat Chem B 2014, 2:577-584].

Coatings have been described for reducing biofilm formation. In one example, a polymeric antifouling coating was based on aggregation of a short amphiphilic four armed PEG-dopamine polymer into particles and on surface binding by catechol chemistry [Mizrahi et al., Langmuir 2013, 29:10087-10094]. Another example is surface grafting of a brush of poly(oligoethylene glycol methyl ether acrylate), which was reported to improve the antifouling properties of poly(2-hydroxyethyl methacrylate-co-methyl methacrylate) [Bozukova et al., Langmuir 2008, 24:6649-6658].

Contact lenses frequently adsorb proteins from the eye fluid, which limits their long-term use. The accumulated protein can lead to discomfort and eye infection, as bacterial adhesion to soft contact lenses and biofilm formation have been reported to be associated with keratitis [Bourcier et al., Br J Ophthalmol 2003, 87:834-838; Tam et al., Invest Ophthalmol Vis Sci 2010, 51:3100-3106].

Tran et al. [Cont Lens Anterior Eye 2012, 35:155-162] describes an attempt o modulate adhesion of *P. aeruginosa* to silicone-hydrogel contact lenses by enhancing wettability of the lens surface using poloxamer or ethylene oxide-butylene oxide surfactants. The surfactants were reported to enhance wettability, but no correlation was found between *P. aeruginosa* adhesion and contact lens wettability.

International Patent Application PCT/IL2014/050604 describes hydrogels with liposomes dispersed therein, which exhibit a reduced friction coefficient compared to neat hydrogels.

U.S. Provisional Patent Application 62/012,379 describes aqueous solutions comprising an ionic polymer and liposomes, for rinsing and/or immersing therein a contact lens, and/or for reducing a friction coefficient of a surface. U.S. Provisional Patent Application 62/012,379 further describes methods of reducing a friction coefficient of a surface by attaching an ionic polymer to the surface and contacting the ionic polymer with liposomes, thereby coating the surface with an amphiphilic lipid.

International Patent Application Publication WO 2008/038292 and U.S. Patent Application Publication No. 20100098749 describe a use of liposomes of several phospholipids above their liquid-crystalline-phase to gel-phase transition temperature (Tm) for lubrication of joints.

Additional background art includes Chen et al. [Polymer 2010, 51:5283-5293].

US 2011052656 and US 7824557 relate to a process which increases the efficiency and effectiveness of introducing antimicrobial compounds into complex biofilm matrices through the use of liposome carriers, thereby removing the biofouling in industrial water bearing systems, including piping, heat exchanges, condensers, filtration systems and fluid storage tanks. Antimicrobial compound containing liposomes are added to water systems prone to biofouling and biofilm formation. The liposomes, being similar in composition to microbial membranes or cells, are readily incorporated into the existing biofilm. Once the antimicrobial compound containing liposomes become entrained with the biofilm matrix, the decomposition or programmed disintegration of the liposome proceeds. Thereafter, the biocidal aqueous core is released to react directly with the biofilm encased microorganisms. Upon the death of the organisms, the polysaccharide/protein matrix decomposes and thereby results in reduced fouling of the water bearing system, resulting in increased heat transfer, increased flux, less deposit of colloidal and particulate solids and dissolved organics on the surface of the microfiltration membrane, thereby reducing the frequency and duration of the membrane cleaning and ultimate replacement.

US6132765 relates to a vehicle for effecting drug delivery from a solid substrate. Hydrogels loaded with liposomal therapeutic agents such as antibiotics are covalently bonded to the surface of substrates such as in-dwelling medical devices, such as implants, catheters. It is particularly useful in the treatment and prevention of biofilm mediated infection often associated with the use of in-dwelling medical devices.

PETER KINGSHOTT ET AL, discloses "Surfaces that resist bioadhesion", CURRENT OPINION IN SOLID STATE AND MATERIALS SCIENCE, GB, (19990801), vol. 4, no. 4, doi:10.1016/S1359-0286(99)00018-2, ISSN 1359-0286, pages 403 - 412.

US 2010/239651 relates to a biodelivery system which increases the efficiency and effectiveness of introducing antimicrobial compounds into complex biofilm matrices through the use of liposome carriers, thereby removing the biofouling in industrial water bearing systems, including piping, heat exchanges, condensers, filtration systems and fluid storage tanks. Antimicrobial compound containing liposomes are added to water systems prone to biofouling and biofilm formation. The liposomes, being similar in composition to microbial membranes or cells, are readily incorporated into the existing biofilm. Once the antimicrobial compound containing liposomes become entrained with the biofilm matrix, the decomposition or disintegration of the liposome proceeds. Thereafter the biocidal core is released to react directly with the biofilm encased microorganisms. Upon the death of the organisms, the matrix decomposes and thereby results in reduced fouling of the water bearing system, resulting in increased heat transfer, increased flux, less deposit of colloidal and particulate solids and dissolved organics on the surface of the microfiltration membrane, thereby reducing the frequency and duration of the membrane cleaning and ultimate replacement.

ANNE DANION ET AL, relates to "Antibacterial activity of contact lenses bearing surface-immobilized layers of intact liposomes loaded with levofloxacin", JOURNAL OF PHARMACEUTICAL SCIENCES, WASHINGTON, US, (20070101), vol. 96, no. 9, doi:10.1002/jps.20871, ISSN 0022-3549, pages 2350 - 2363.

BUDAI ET AL, relates to "Gels and liposomes in optimized ocular drug delivery: Studies on ciprofloxacin formulations", INTERNATIONAL JOURNAL OF PHARMACEUTICS, ELSEVIER BV, NL, (20070830), vol. 343, no. 1-2, doi:10.1016/J.IJPHARM.2007.04.013, ISSN 0378-5173, pages 34 - 40.

XINMING L ET AL, relates to "Polymeric hydrogels for novel contact lens-based ophthalmic drug delivery systems: A review", CONTACT LENS AND ANTERIOR EYE, STOCKTON PRESS, BASINGSTOKE, GB, vol. 31, no. 2, doi:10.1016/J.CLAE.2007.09.002, ISSN 1367-0484, (20080401), pages 57 - 64.

HU, X.; HAO, L.; WANG, H. ET AL., relates to "Hydrogel Contact lens for extended delivery of ophthalmic drugs", INTERNATIONAL JOURNAL OF POLYMER SCIENCES, (2011), vol. 2011, pages 1 - 9.

US4348329 relates to new phospholipids containing a conjugated di-yne system, prepared by esterification and/or etherification of glycerol by conventional techniques. The conjugated di-yne systems can be cross-linked by actinic radiation to give intermolecular and, in certain cases, also intramolecular cross-linking. Thin layers of the phospholipids can be coated onto substrates subsequently to be brought into contact with blood or other body fluids and then cross-linked to give polymer coated surfaces which have a reduced tendency to initiate blood coagulation, for example, compared to untreated surfaces. Artificial lenses for the human eye treated in this way have a reduced tendency to cause endothelial damage. The phospholipids can also be formed into liposomes and the conjugated di-yne system then cross-linked. Physiologically active materials such as drugs, enzymes or antigens can be incorporated into the liposomes which can then be used as prolonged release formulations.

EP 0319638 relates to a composition and method for restoring the skin's natural barrier to moisture loss, thereby enhancing the ability of the skin to retain its moisture. The composition comprises a liposome, wherein the liposome comprises a cerebroside, a phospholipid and a cholesteric material. There is further disclosed cosmetic compositions and methods for treating the skin comprising a multiple barrier delivery system for applying a biologically active material to the skin, the delivery system comprising a liposome, an encapsulated biologically active material, and a starch-grafted polyacrylic acid gel.

US 6103246 relates to a composition which is a cream or lotion for topical application to human skin. The composition includes emu oil as a component. Emu oil is generally comprised of myristic acid, palmitic acid, palmitoleic acid, margaric acid, stearic acid, elaidic acid, oleic acid, vaccenic acid, linoleic acid, linolenic acid, arachidic acid and eicosenoic acid. The composition also includes a medically active component, such as lactic acid or some other alpha-hydroxy acid. The balance of the components of the composition are water, glycerin, and an emulsion base. Liposomes may be included in the composition. The composition provides for increased skin penetrability due to the emu oil.

VERBERNE G ET AL, relates to "Liposomes as potential biolubricant additives for wear reduction in human synovial joints", WEAR, ELSEVIER SEQUOIA, LAUSANNE, CH, vol. 268, no. 7-8, doi:10.1016/J.WEAR.2009.12.037, ISSN 0043-1648, (20100309), pages 1037 - 1042.

XU J ET AL, relates to "Cyclodextrin-containing hydrogels for contact lenses as a platform for drug incorporation and release", ACTA BIOMATERIALIA, ELSEVIER, AMSTERDAM, NL, vol. 6, no. 2, ISSN 1742-7061, (20100201), pages 486 - 493.

DANION, A.; BROCHU, H. ET AL., relates to "Fabrication and characterization of contact lenses bearing surface-immobilized layers of intact liposomes", J BIOMED MATER RES A, (20070130), pages 41 - 51.

US 2009/039035 relates to a process which increases the efficiency and effectiveness of introducing antimicrobial compounds into complex biofilm matrices through the use of liposome carriers, thereby removing the biofouling in industrial water bearing systems, including piping, heat exchanges, condensers, filtration systems and fluid storage tanks. Antimicrobial compound containing liposomes are added to water systems prone to biofouling and biofilm formation. The liposomes, being similar in composition to microbial membranes or cells, are readily incorporated into the existing biofilm. Once the antimicrobial compound containing liposomes become entrained with the biofilm matrix, the decomposition or programmed disintegration of the liposome proceeds.; Thereafter the biocidal aqueous core is released to react directly with the biofilm encased microorganisms. Upon the death of the organisms, the polysaccharide/protein matrix decomposes and thereby results in reduced fouling of the water bearing system, resulting in increased heat transfer, increased flux, less deposit of colloidal and particulate solids and dissolved organics on the surface of the microfiltration membrane, thereby reducing the frequency and duration of the membrane cleaning and ultimate replacement.

### SUMMARY OF THE INVENTION

The present invention relates to a method of inhibiting adsorption of a biofouling-promoting agent on a surface of a substrate, said substrate being composed of a hydrogel, wherein the method does not comprise a method of treatment of a human or animal body, the method comprising contacting the substrate with a composition which comprises liposomes and a polymer, wherein said liposomes comprise at least one phospholipid, said at least one phospholipid comprising at least one phosphatidylcholine, and a concentration of phospholipids in said liposomes in said composition is in a range of from 1 mM to 150 mM, wherein said liposomes are characterized by a melting point below a temperature of said surface during said contacting, and wherein said polymer is selected from the group consisting of hyaluronic acid and hydroxypropyl methyl cellulose.

The present invention relates also to a method of inhibiting biofilm formation on a surface of a substrate, said substrate being composed of a hydrogel, wherein the method does not comprise a method of treatment of a human or animal body, the method comprising contacting the substrate with a composition which comprises liposomes and a polymer, wherein said liposomes comprise at least one phospholipid, said at least one phospholipid comprising at least one phosphatidylcholine, and a concentration of phospholipids in said liposomes in said composition is in a range of from 1 mM to 150 mM, wherein said liposomes are characterized by a melting point below a temperature of said surface during said contacting, and wherein said polymer is selected from the group consisting of hyaluronic acid and hydroxypropyl methyl cellulose.

Preferably, said contacting comprises incorporating said composition which comprises liposomes within said hydrogel.

Preferably, said substrate is prepared by forming said hydrogel in the presence of said liposomes.

Preferably, said composition further comprises a hydrogel.

Preferably, said biofouling-promoting agent is selected from the group consisting of a biofouling-promoting protein and a biofouling-promoting polysaccharide.

Preferably, said substrate is a contact lens, the method comprising rinsing and/or immersing the contact lens in a solution comprising said liposomes, said polymer, and an aqueous carrier.

### BRIEF DESCRIPTION OF THE SEVERAL VIEWS OF THE DRAWINGS

Some aspects of the disclosure are herein described, by way of example only, with reference to the accompanying drawings. With specific reference now to the drawings in detail, it is stressed that the particulars shown are by way of example and for purposes of illustrative discussion of aspects of the disclosure. In this regard, the description taken with the drawings makes apparent to those skilled in the art how aspects of the disclosure may be practiced.

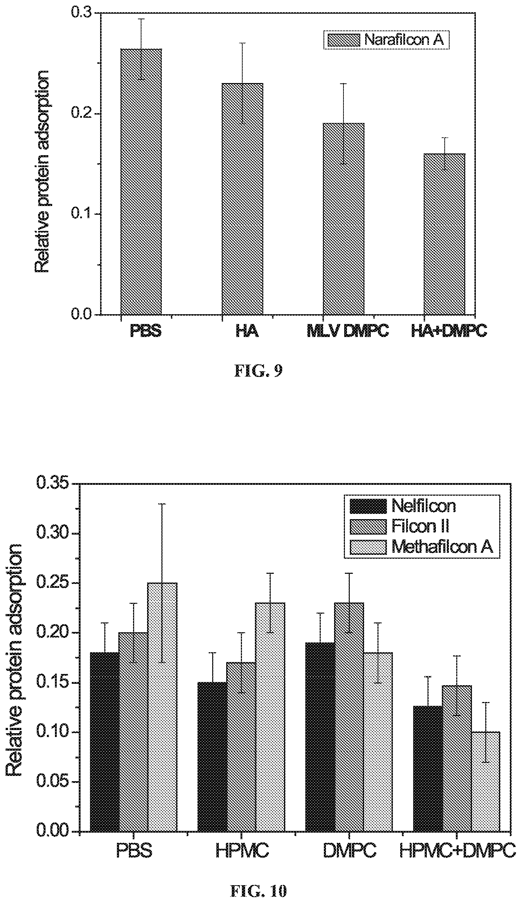

In the drawings:
Only HA+DMPC of figure 9 and HPMC + DMPC of figure 10 fall under the scope of protection.

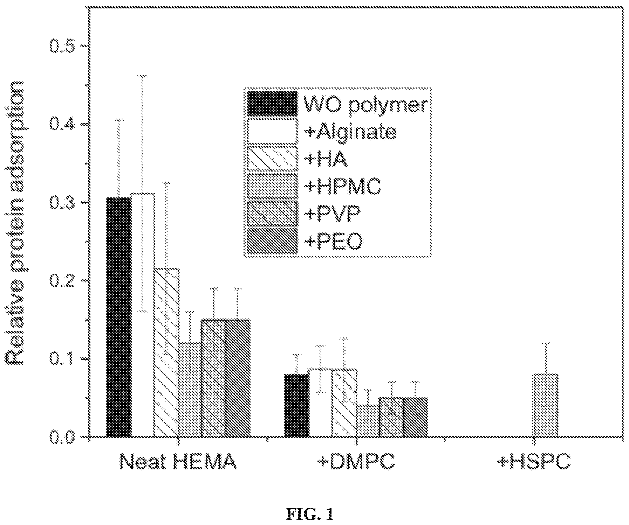

FIG. 1 is a bar graph showing the relative adsorption of a protein (anti-IgG antibody) to a cross-linked poly(2-hydroxyethyl methacrylate) hydrogel (Neat HEMA) and to cross-linked poly(2-hydroxyethyl methacrylate) hydrogels loaded with DMPC (dimyristoyl phosphatidylcholine) liposomes (+DMPC) or HSPC (hydrogenated soy phosphatidylcholine) liposomes (+HSPC), as well as with alginate, hyaluronic acid (HA), hydroxypropyl methyl cellulose (HPMC), polyvinylpyrrolidone (PVP) or polyethylene oxide (PEO), or without a polymer loaded into the hydrogel (WO polymer) (normalized to adsorption to a tissue culture polystyrene surface);

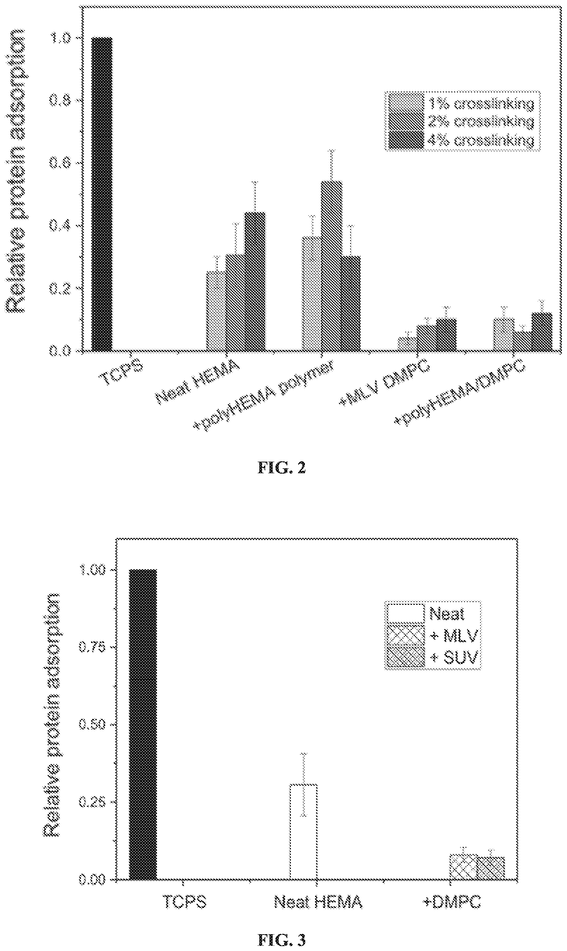

FIG. 2 is a bar graph showing the relative adsorption of a protein (anti-IgG antibody) to a poly(2-hydroxyethyl methacrylate) hydrogel (Neat HEMA) cross-linked with 1 %, 2 % or 4 % EDGMA (ethylene glycol dimethacrylate), and to poly(2-hydroxyethyl methacrylate) hydrogels cross-linked with 1 %, 2 % or 4 % EDGMA and loaded with non-cross-linked poly(2-hydroxyethyl methacrylate) (+polyHEMA polymer), with DMPC (dimyristoyl phosphatidylcholine) multilamellar vesicles (+MLV DMPC), and with DMPC multilamellar vesicles mixed with non-cross-linked poly(2-hydroxyethyl methacrylate) (+polyHEMA/DMPC) (normalized to adsorption to a tissue culture polystyrene (TCPS) surface);
FIG. 3 is a bar graph showing the relative adsorption of a protein (anti-IgG antibody) to a cross-linked poly(2-hydroxyethyl methacrylate) hydrogel (Neat HEMA) and to cross-linked poly(2-hydroxyethyl methacrylate) hydrogels loaded with DMPC (dimyristoyl phosphatidylcholine) (+DMPC) in the form of multilamellar vesicles (+MLV) or small unilamellar vesicles (+SUV) (normalized to adsorption to a tissue culture polystyrene (TCPS) surface);

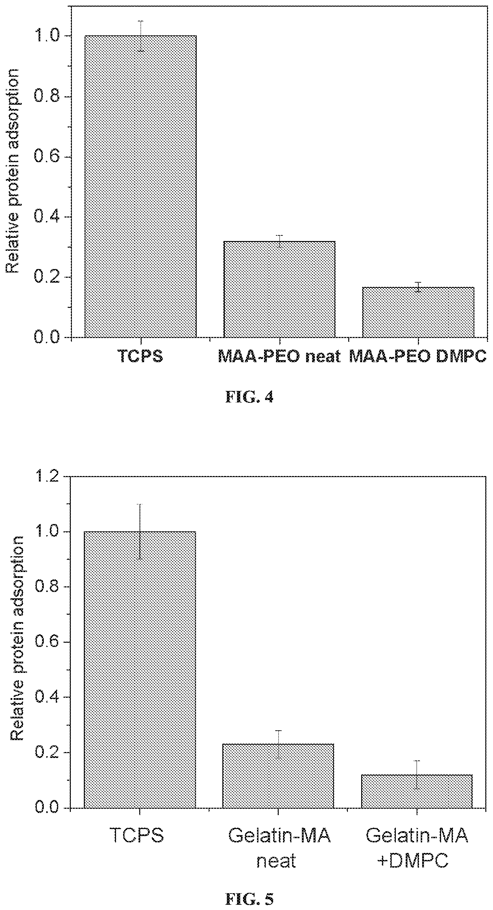

FIG. 4 is a bar graph showing the relative adsorption of a protein (anti-IgG antibody) to a methacrylic acid-polyethylene oxide hydrogel (MAA-PEO neat) and to a methacrylic acid-polyethylene oxide hydrogel loaded with dimyristoyl phosphatidylcholine liposomes (MAA-PEO DMPC) (normalized to adsorption to a tissue culture polystyrene (TCPS) surface);
FIG. 5 is a bar graph showing the relative adsorption of a protein (anti-IgG antibody) to a gelatin methacrylate hydrogel (Gelatin-MA neat) and to a gelatin methacrylate hydrogel loaded with dimyristoyl phosphatidylcholine liposomes (Gelatin-MA+DMPC) (normalized to adsorption to a tissue culture polystyrene (TCPS) surface);

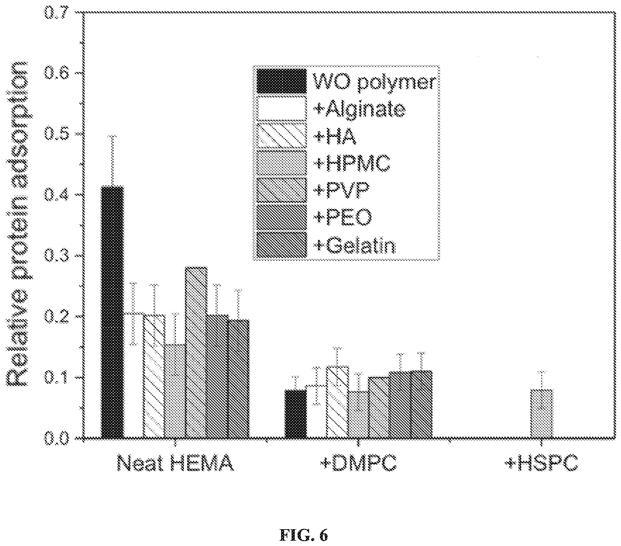

FIG. 6 is a bar graph showing the relative adsorption of a protein (anti-IgG antibody) to a cross-linked poly(2-hydroxyethyl methacrylate) hydrogel (Neat HEMA) and to cross-linked poly(2-hydroxyethyl methacrylate) hydrogels loaded with DMPC (dimyristoyl phosphatidylcholine) liposomes (+DMPC) or HSPC (hydrogenated soy phosphatidylcholine) liposomes (+HSPC), as well as with alginate, hyaluronic acid (HA), hydroxypropyl methyl cellulose (HPMC), polyvinylpyrrolidone (PVP), polyethylene oxide (PEO) or gelatin, or without a polymer loaded into the hydrogel (WO polymer) (normalized to adsorption to a tissue culture polystyrene surface; hydrogels were completely dried at 37 °C and then rehydrated in PBS prior to protein adsorption);

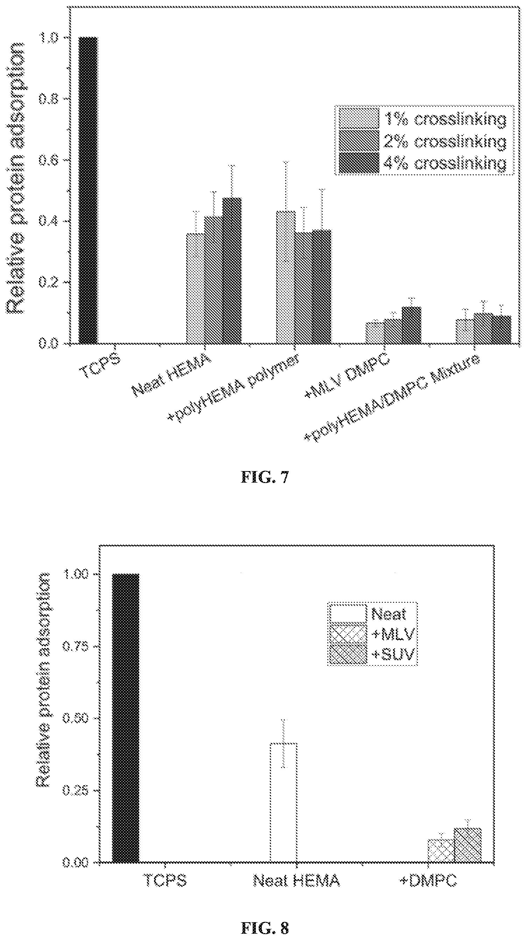

FIG. 7 is a bar graph showing the relative adsorption of a protein (anti-IgG antibody) to a poly(2-hydroxyethyl methacrylate) hydrogel (Neat HEMA) cross-linked with 1 %, 2 % or 4 % EDGMA (ethylene glycol dimethacrylate), and to poly(2-hydroxyethyl methacrylate) hydrogels cross-linked with 1 %, 2 % or 4 % EDGMA and loaded with non-cross-linked poly(2-hydroxyethyl methacrylate) (+polyHEMA polymer), with DMPC (dimyristoyl phosphatidylcholine) multilamellar vesicles (+MLV DMPC), and with DMPC multilamellar vesicles mixed with non-cross-linked poly(2-hydroxyethyl methacrylate) (+polyHEMA/DMPC Mixture) (normalized to adsorption to a tissue culture polystyrene (TCPS) surface; hydrogels were completely dried at 37 °C and then rehydrated in PBS prior to protein adsorption);
FIG. 8 is a bar graph showing the relative adsorption of a protein (anti-IgG antibody) to a cross-linked poly(2-hydroxyethyl methacrylate) hydrogel (Neat HEMA) and to cross-linked poly(2-hydroxyethyl methacrylate) hydrogels loaded with DMPC (dimyristoyl phosphatidylcholine) (+DMPC) in the form of multilamellar vesicles (+MLV) or small unilamellar vesicles (+SUV) (normalized to adsorption to a tissue culture polystyrene (TCPS) surface; hydrogels were completely dried at 37 °C and then rehydrated in PBS prior to protein adsorption);
FIG. 9 is a bar graph showing the relative adsorption of a protein (anti-IgG antibody) to narafilcon A contact lenses following treatment by being immersed in PBS or in a solution of hyaluronic acid (HA), dimyristoyl phosphatidylcholine liposomes (MLV DMPC), or both hyaluronic acid and dimyristoyl phosphatidylcholine liposomes (HA+DMPC); and
FIG. 10 is a bar graph showing the relative adsorption of a protein (anti-IgG antibody) to nelfilcon, filcon II and methafilcon A contact lenses following treatment by being immersed in PBS or in a solution of hydroxypropyl methyl cellulose (HPMC), dimyristoyl phosphatidylcholine liposomes (DMPC), or both hydroxypropyl methyl cellulose and dimyristoyl phosphatidylcholine liposomes (HPMC+DMPC).

### DESCRIPTION OF SPECIFIC ASPECTS OF THE DISCLOSURE

The present disclosure, in some aspects thereof, relates to material science and, more particularly, but not exclusively, to compositions and methods for reducing biofilm formation on a surface of various substrates.

As discussed hereinabove, the formation of biofilms presents a formidable challenge in medicine and other practices.

The present inventors have now surprisingly uncovered that combining liposomes and hydrogel-containing matrix reduced absorption of biofouling-promoting agents and thus imparts anti-biofouling (ABF) properties to the matrix. The present inventors have therefore envisioned that including liposomes in a hydrogel-containing substrate and/or using a composition composed of liposomes and a hydrogel on non-hydrogel substrates can be used for reducing the absorption of biofouling-promoting agents to the surface of such substrates and to inhibit biofilm formation on such surfaces. These findings represent a novel solution of the challenge of inhibiting biofilm formation on a variety of articles, such as medical, agricultural and veterinary articles.

As demonstrated in the Examples section that follows, treatment of substrates with liposomes was surprisingly found to considerably reduce adsorption of biofouling-promoting agents such as proteins, in a simple and efficient manner. Protein adsorption is associated with biofilm formation because protein adsorption can create a conditioning film on a substrate which facilitates cell attachment, and/or because proteins are an important component of cells.

Referring now to the drawings, FIGs. 1-8 show that loading various hydrogels with liposomes surprisingly inhibits protein adsorption to the hydrogel surface. FIGs. 9 and 10 show that simple immersion of contact lenses in a solution comprising liposomes inhibits protein adsorption to the contact lens surface.

In addition, FIGs. 1, 9 and 10 further show that addition of a polymer to liposomes in the hydrogel or solution surprisingly enhances the inhibition of protein adsorption. Such an effect is particularly surprising, as it would have been expected that a surface comprising liposome lipids and a polymer would have properties intermediate between those of a surface comprising lipids alone and a surface comprising the polymer alone, rather than anti-adsorption properties superior to those of either the lipid-comprising surface and the polymer-comprising surface.

These results indicate that contact of a substrate with liposomes can efficiently reduce adsorption to a surface, as well as biofouling. The results further indicate that the effect of the liposomes can optionally be enhanced by use of the liposomes in combination with a polymer. It is to be appreciated that such methodology is simple to perform and efficacy was demonstrated for liposomes and polymers which are naturally occurring and/or recognized as non-toxic. Thus, the methodology can advantageously be utilized in a wide variety of applications, including applications wherein the substrate is implantable or otherwise contacts a body.

Without being bound by any particular theory it is believed that lipids from the liposomes promote the formation of hydration layers at the surface, which serves as a barrier to adsorption on the surface. It is further believed that inclusion of liposomes in a hydrogel results in lipids reaching the surface by diffusion, and that immersion of a substrate (e.g., contact lens) in a solution comprising liposomes results in lipids from the solution becoming adhered to the surface.

Based on the results presented herein, inhibition of adsorption and biofilm formation on of a wide variety of substrates may be effected, in accordance with various aspects of the disclosure described herein.

### Inhibition of adsorption:

According to an aspect of the disclosure, there is provided a method of inhibiting adsorption of a biofouling-promoting agent on a surface of a substrate. The method, according to some aspects of the present disclosure, is effected by contacting the substrate with a composition which comprises liposomes.

Herein, the term "contacting" encompasses effecting any form of physical contact between two or more substances (e.g., a substrate and composition described herein), including mixing, incorporating and/or embedding one substance within another, and contact only at an interface between the substances. The term "contacting" further encompasses forming one or more of the substances in a manner such that it is in contact with the other substance(s) as soon as it is formed, as well as contacting preformed substances.

The term "biofouling-promoting agent", as used herein throughout, refers to an agent whose presence facilitates formation and/or participates in formation of a biofilm (as defined herein) on a substrate surface. An agent is considered to facilitate formation of a biofilm on a substrate surface when a presence of the agent enhances formation of a biofilm on a substrate surface as compared to formation of a biofilm on the same substrate surface in an absence of the agent. An agent is considered to participate in formation of a biofilm on a substrate surface when the biofilm formed on the surface comprises the agent as a portion of the biofilm.

In some aspects described herein, an agent is identified as a biofouling-promoting agent by comparing growth (e.g., over the course of 1, 2, 3, 4, 5, 6 or 7 days) of a biofilm (e.g., *P. aeruginosa*) on the surface in the presence of an aqueous liquid (e.g., water or broth, optionally at 37 °C) and the agent, to growth of a biofilm (under the same conditions) on the surface in the presence of the same aqueous liquid (e.g., water or broth) without the agent. The agent is optionally mixed within the aqueous liquid, or alternatively, adsorbed onto the surface prior to exposure of the surface to the aqueous liquid. The growth of the biofilm is considered as the biofilm load at the end of the growth period (e.g., 1, 2, 3, 4, 5, 6 or 7 days) minus the initial biofilm load. Optionally, the measurement is performed such that the initial biofilm is zero (e.g., absent or at least undetectable), for example, the microorganism is in a planktonic form, such that growth of the biofilm is considered as the biofilm load at the end of the growth period. In some aspects described herein, biofilm load is defined as an area of the biofilm.

In some aspects described herein, biofilm load is defined as a mass and/or volume of the biofilm.

In some aspects described herein, biofilm load is defined as a number of cells in the biofilm.

The biofilm load may optionally be determined using any technique known in the art for detecting and quantifying an amount of cells and/or microorganisms in a biofilm.

In some of these aspects, an agent is considered a biofouling-promoting agent if the growth of a biofilm in its presence is at least 10 % higher than growth of a biofilm in the absence of the agent.

In some of these aspects, an agent is considered a biofouling-promoting agent if the growth of a biofilm in its presence is at least 20 % higher than growth of a biofilm in the absence of the agent.

In some of these aspects, an agent is considered a biofouling-promoting agent if the growth of a biofilm in its presence is at least 50 % higher than growth of a biofilm in the absence of the agent.

In some of these aspects, an agent is considered a biofouling-promoting agent if the growth of a biofilm in its presence is at least 100 % higher than (i.e., two-fold) the growth of a biofilm in the absence of the agent.

Examples of biofouling-promoting agents include, without limitation, a biofouling-promoting protein and a biofouling-promoting polysaccharide, that is, any protein or polysaccharide which is a biofouling-promoting agent as defined herein.

In some aspects described herein, the biofouling-promoting agent is a protein.

In some aspects described herein, the method is considered as being capable of inhibiting adsorption of a biofouling-promoting agent when the method is capable of inhibiting adsorption of a selected biofouling-promoting agent (e.g., the selected agent is considered representative of biofouling-promoting agents in general). In some aspects, the selected biofouling-promoting agent is a protein. In some aspects, the selected protein is an antibody which does not exhibit any specific affinity to the substrate (e.g., an anti-IgG antibody, as exemplified herein).

The term "biofilm", as used herein throughout, refers to an aggregate of living cells which are stuck to each other and/or immobilized onto a surface as colonies. The cells are frequently embedded within a self-secreted matrix of extracellular polymeric substance (EPS), also referred to as "slime", which is a polymeric sticky mixture of nucleic acids, proteins and polysaccharides.

In the context of the present aspects, the living cells forming a biofilm can be cells of a unicellular microorganism, including prokaryotes (e.g., bacteria, archaeal microorganisms) and eukaryotes such as fungi and protists (e.g., algae, euglena, protozoa, dinoflagellates, apicomplexa, trypanosomes, amoebae); or cells of multicellular organisms, in which case the biofilm can be regarded as a colony of cells (as in the case of the unicellular organisms) or as a lower form of a tissue.

According to some aspects of the present disclosure, the cells are of microorganism origins, and the biofilm is a biofilm of microorganisms, such as bacteria, archaeal microorganisms, protists and fungi. The cells of a microorganism growing in a biofilm are typically physiologically distinct from cells in the "planktonic" form of the same organism, which by contrast, are single cells that may float or swim in a liquid medium.

The term "substrate" as used herein, is as described herein, and further refers to any surface, structure, product or material which can support, harbor or promote the growth of a microorganism. The substrate is optionally a portion of an object (e.g., an article of manufacture) which can support, harbor or promote the growth of a microorganism. Such a portion of an object may span only a portion of an area of the object, such that a surface of the substrate represents only a portion of a surface of the object (e.g., a portion most likely to support, harbor or promote the growth of a microorganism); and/or span only a portion of the thickness of the object (e.g., along an axis perpendicular to a surface of the substrate and object), such that the substrate does not include the entire volume of the object lying underneath a surface of the substrate (which may represent the entire surface of the object or only a portion of the surface of the object). Non-limiting examples include the inner walls of a storage container (e.g., a box, a can) and/or conduit (e.g., a tubes, a pipe) for an organic product susceptible to spoilage associated with biofouling, for example, food and/or drink (e.g., a food container, a water pipe), surfaces intended to come into contact with such an organic product (e.g., agricultural and/or food processing machinery, a kitchen surface, water-purification equipment), and surfaces exposed to moisture (e.g., bathroom walls, water system components, outer surfaces of housing exposed to rain, surfaces in the vicinity of water leakage).

In some aspects, the substrate is a medical device or any other device which is intended for contacting a living tissue, as defined herein.

As used herein, the phrase "medical device" includes any material or device that is used on, in, or through a subject's body, for example, in the course of medical treatment (e.g., for a disease or injury). The subject may be human or a non-human animal, such that the phrase "medical device" encompasses veterinary devices. Medical devices include, but are not limited to, such items as medical implants (including permanent implants and transient implants), wound care devices, drug delivery devices, contact lenses and body cavity and personal protection devices. The medical implants include, but are not limited to, catheters (e.g., urinary catheters, intravascular catheters), injection ports, intubation equipment, dialysis shunts, wound drain tubes, skin sutures, vascular grafts, implantable meshes, intraocular devices, heart valves. Wound care devices include, but are not limited to, general wound dressings, biologic graft materials, tape closures and dressings, and surgical incise drapes. Drug delivery devices include, but are not limited to, needles, drug delivery skin patches, drug delivery mucosal patches and medical sponges. Body cavity and personal protection devices, include, but are not limited to, tampons, sponges, surgical and examination gloves, and toothbrushes. Birth control devices include, but are not limited to, intrauterine devices (IUDs), diaphragms and condoms.

In some aspects described herein, the inhibition of adsorption described herein is for reducing adhesion of pathogenic microorganisms (e.g., any biofilm-forming microorganism described herein which is potentially pathogenic) to a medical device.

In some aspects described herein, adsorption of the biofouling-promoting agent (any biofouling-promoting agent described herein) on the surface of the substrate subjected to a method described herein (according to any of the respective aspects) is reduced by at least 10 % relative to adsorption on the surface of the substrate in the absence of the composition which comprises liposomes In some aspects, adsorption is reduced by at least 20 %. In some aspects, adsorption is reduced by at least 30 %. In some aspects, adsorption is reduced by at least 40 %. In some aspects, adsorption is reduced by at least 50 %. In some aspects, adsorption is reduced by at least 60 %. In some aspects, adsorption is reduced by at least 70 %. In some aspects, adsorption is reduced by at least 80 %. In some aspects, adsorption is reduced by at least 90 %.

Reduction of an amount of adsorbed biofouling-promoting agent may optionally be determined using any technique known in the art for detecting and quantifying an amount of agent, including, without limitation, using a labeled biofouling-promoting agent (e.g., as exemplified herein in the Examples section). The reduction is optionally measured by contacting each of the aforementioned surfaces (e.g., for 2 hours) with an aqueous solution (optionally comprising phosphate buffer, e.g., 0.1 M phosphate) of the biofouling-promoting agent (e.g., at 37 °C and/or pH 7), followed by repeated rinses to remove non-adsorbed agent (e.g., as exemplified in the examples section herein). The concentration of the biofouling-promoting agent in the aqueous solution is optionally 1 µg/ml or the concentration of a saturated solution of the agent, whichever concentration is lower.

According to an aspect of the disclosure, there is provided a composition comprising liposomes (according to any of the respective aspects described herein), identified for use in inhibiting adsorption of a biofouling-promoting agent on a surface of a substrate (e.g., in accordance with any aspect of a respective method described herein).

In some aspects described herein, the composition comprising liposomes comprises a hydrogel (according to any of the respective aspects described herein).

In some aspects described herein, the composition comprising liposomes is a liquid comprising the liposomes. In some aspects the liquid is an aqueous liquid (e.g., liposomes in water or in an aqueous solution).

In some aspects described herein, contacting the substrate with a composition comprising liposomes is effected by applying a composition comprising a hydrogel (according to any of the respective aspects described herein) and/or a composition which is a liquid (according to any of the respective aspects described herein) onto a surface of the substrate.

In some aspects, applying a composition onto a surface of the substrate is effected by rinsing and/or immersing at least a portion of the substrate in the composition. In some such aspects, the composition is a liquid (according to any of the respective aspects described herein).

Herein throughout, the term "rinsing" generally refers to brief contact (e.g., for several seconds) with a liquid, whereas the term "immersing" generally refers to longer periods of contact with a liquid. However, both terms refer to contact with a liquid, and are typically used herein together to encompass all forms of contact with a liquid, for example, in aspects wherein the difference between rinsing and immersing is of no particular significance.

According to an aspect of the disclosure, there is provided an article comprising a substrate (as defined herein) and the composition identified for use in inhibiting adsorption of a biofouling-promoting agent (according to any of the respective aspects described herein), the composition being applied on a surface of the substrate.

In some aspects described herein, the article consists of the substrate and the composition applied on a surface thereof.

In some aspects described herein, the substrate and the composition applied on a surface thereof form only a portion of the article.

In some aspects described herein, the substrate and the composition applied on a surface thereof form a structural component of the article. In some aspects, the substrate and the composition applied on a surface thereof form only a portion of the article. In some aspects, the substrate and the composition applied on a surface thereof are manufactured as a unit which is assembled with other units to form the article.

In some aspects described herein, the article is a medical device (the substrate being a medical device or portion of a medical device, as described herein). In some aspects, the medical device is a device designed to come into contact with a part of the body susceptible to infection, such as an internal portion of the body, a mucous membrane and/or a surface of the eye. Examples of such medical devices include, without limitation, surgical tools and implants (which are for coming into contact with an internal portion of the body) and contact lenses (which are for contacting a surface of the eye).

Exemplary articles include the following:
Medical devices such as, but are not limited to, pacemakers, heart valves, replacement joints, catheters, catheter access ports, dialysis tubing, gastric bands, shunts, screw plates, artificial spinal disc replacements, internal implantable defibrillators, cardiac resynchronization therapy devices, implantable cardiac monitors, mitral valve ring repair devices, left ventricular assist devices (LVADs), artificial hearts, implantable infusion pumps, implantable insulin pumps, stents, implantable neurostimulators, maxillofacial implants, dental implants,;
Packages or containers, for example, food packages and containers, beverage packages and containers, medical device packages, agricultural packages and containers (of agrochemicals), blood sample or other biological sample packages and containers, and any other packages or containers of various articles;
Food packages such as packages of dairy products and/or containers for storage or transportation of dairy products;
Milk storage and processing devices such as, but not limited to, containers, storage tanks, raw milk holding equipments, dairy processing operations conveyer belts, tube walls, gaskets, rubber seals, stainless steel coupons, piping systems, filling machine, silo tanks, heat exchangers, post-pasteurization equipment, pumps, valves, separators, and spray devices;
Energy harvesting device, for example, a microelectronic device, a microelectromechanical device, a photovoltaic device;
Microfluidic devices, for example, micropumps or micro valves;
Sealing parts, for example, O rings, and the like;
Articles having a corrodible surface;
Agricultural devices, as, for example, described herein;
Textiles, for example, tough cottons;
Fuel transportation devices;
Construction elements, such as, but not limited to, paints, walls, windows, door handles;
Elements of water treatment systems (such as for containing and/or transporting and/or treating aqueous media or water), devices, containers, filters, tubes, solutions and gases; and
Elements of organic waste treatment systems (such as for containing and/or disposing and/or transporting and/or treating organic waste), devices, containers, filters, tubes, solutions and gases.

In some aspects described herein, the article is characterized in that upon contact with the agent under biofouling-promoting conditions, a biofilm load on the substrate is lowered by at least 10 % compared to a biofilm load on the substrate when being devoid of the composition (upon contact with the same agent under the same conditions). In some aspects, the biofilm load is reduced by at least 20 %. In some aspects, the biofilm load is reduced by at least 30 %. In some aspects, the biofilm load is reduced by at least 40 %. In some aspects, the biofilm load is reduced by at least 50 %. In some aspects, the biofilm load is reduced by at least 60 %. In some aspects, the biofilm load is reduced by at least 70 %. In some aspects, the biofilm load is reduced by at least 80 %. In some aspects, the biofilm load is reduced by at least 90 %.

Herein throughout, the term "biofilm-promoting conditions" refers to conditions suitable for formation and growth of a biofilm of a cell (e.g., *P. aeruginosa*), for example, wherein a surface is in contact (e.g., over the course of 1, 2, 3, 4, 5, 6 or 7 days) with an aqueous liquid (e.g., water or broth, optionally at 37 °C) containing such cells.

In some aspects described herein, biofilm load is defined as an area of the biofilm.

In some aspects described herein, biofilm load is defined as a mass and/or volume of the biofilm.

In some aspects described herein, biofilm load is defined as a number of cells in the biofilm.

The biofilm load may optionally be determined using any technique known in the art for detecting and quantifying an amount of cells and/or microorganisms in a biofilm.

In some aspects described herein, the time period of biofilm formation, after which biofilm load is determined, is in determined in accordance with the biofilm load, for example, the time period being a time period after which a biofilm covers 100 %, 50 %, or any other pre-determined percentage of the area of the substrate in the absence of inhibition of biofilm formation by contact with a composition comprising liposomes. For example, if a biofilm grows to cover 50 % of a surface in the absence of biofilm formation inhibition, and during the same a time period, a biofilm grows to cover 30 % of a surface in the presence of biofilm formation inhibition, then inhibition of biofilm formation may be considered to result in a reduction of 40 % (i.e., (50 % - 30 %)/50 %) in biofilm formation.

Herein, the phrase "upon contact with the agent" means that in addition to the biofilm-promoting conditions, the agent is also present (e.g., in the aqueous liquid containing the cells).

### Inhibition of biofilm formation:

According to an aspect of the disclosure, there is provided a method of inhibiting biofilm formation on a surface of a substrate (as defined herein in any aspect and any combination of aspects), the method comprising contacting (as described in any of the respective aspects described herein) the substrate with a composition which comprises liposomes.

In some aspects, "inhibiting biofilm formation" refers to the prevention of formation of a biofilm; and/or to a reduction in the rate of buildup of a biofilm; and/or to a reduction in the mass of a biofilm, the area or the volume of the biofilm, or in the number of cells forming the biofilm.

In some aspects described herein, the inhibition of adsorption described herein is for reducing adhesion of pathogenic microorganisms (e.g., any biofilm-forming microorganism described herein which is potentially pathogenic) to a medical device. Such a reduction may result in inhibiting biofilm formation, as defined in some aspects herein.

In some aspects described herein, biofilm formation on the surface of the substrate subjected to a method described herein (according to any of the respective aspects) is reduced by at least 10 % relative to biofilm formation on the surface of the substrate in the absence of the composition which comprises liposomes. In some aspects, biofilm formation is reduced by at least 20 %. In some aspects, biofilm formation is reduced by at least 30 %. In some aspects, biofilm formation is reduced by at least 40 %. In some aspects, biofilm formation is reduced by at least 50 %. In some aspects, biofilm formation is reduced by at least 60 %. In some aspects, biofilm formation is reduced by at least 70 %. In some aspects, biofilm formation is reduced by at least 80 %. In some aspects, biofilm formation is reduced by at least 90 %.

The reduction in biofilm formation is optionally determined by measuring a biofilm load (in accordance with any of the respective aspects described herein) for a biofilm of a cell (e.g., *P. aeruginosa*) on each surface after being subjected to biofouling-promoting conditions, as defined herein (e.g., over the course of 1, 2, 3, 4, 5, 6 or 7 days, or any other time period as described herein).

According to an aspect of the disclosure, there is provided a composition comprising liposomes (according to any of the respective aspects described herein), identified for use in inhibiting biofilm formation on a surface of a substrate (e.g., in accordance with any aspect of a respective method described herein).

In some aspects described herein, , the composition comprising liposomes comprises a hydrogel (according to any of the respective aspects described herein).

In some aspects described herein, the composition comprising liposomes is a liquid comprising the liposomes (according to any of the respective aspects described herein). In some aspects the liquid is an aqueous liquid (e.g., liposomes in water or in an aqueous solution), according to any of the respective aspects described herein.

In some aspects described herein, contacting the substrate with a composition comprising liposomes is effected by applying a composition comprising a hydrogel (according to any of the respective aspects described herein) and/or a composition which is a liquid (according to any of the respective aspects described herein) onto a surface of the substrate.

In some aspects, applying a composition onto a surface of the substrate is effected by rinsing and/or immersing at least a portion of the substrate in the composition. In some such aspects, the composition is a liquid (according to any of the respective aspects described herein).

According to an aspect of the disclosure, there is provided an article comprising a substrate (as defined herein) and the composition identified for use in inhibiting biofilm formation (according to any of the respective aspects described herein), the composition being applied on a surface of the substrate.

In some aspects described herein, the article consists of the substrate and the composition applied on a surface thereof.

In some aspects described herein, the substrate and the composition applied on a surface thereof form a structural component of the article. In some aspects, the substrate and the composition applied on a surface thereof are manufactured as a unit which is assembled with other units to form the article.

In some aspects described herein, the article is a medical device. In some aspects, the medical device is a device designed to come into contact with a part of the body susceptible to infection, such as an internal portion of the body, a mucous membrane and/or a surface of the eye. Examples of such medical devices include, without limitation, surgical tools, implants and contact lenses.

In some aspects described herein, the article is characterized in that under biofouling-promoting conditions, a biofilm load on the substrate is lowered by at least 10 % compared to a biofilm load on the substrate when being devoid of the composition (under the same conditions). In some aspects, the biofilm load is reduced by at least 20 %. In some aspects, the biofilm load is reduced by at least 30 %. In some aspects, the biofilm load is reduced by at least 40 %. In some aspects, the biofilm load is reduced by at least 50 %. In some aspects, the biofilm load is reduced by at least 60 %. In some aspects, the biofilm load is reduced by at least 70 %. In some aspects, the biofilm load is reduced by at least 80 %. In some aspects, the biofilm load is reduced by at least 90 %.

### Hydrogel and liposomes in composition:

According to an aspect of the disclosure, there is provided a method of inhibiting adsorption of a biofouling-promoting agent on a surface of a substrate (in accordance with any of the respective aspects described herein) and/or a method of inhibiting biofilm formation on a surface of a substrate (in accordance with any of the respective aspects described herein), the method comprising contacting the substrate with a composition which comprises liposomes and a hydrogel.

According to an aspect of the disclosure, there is provided a composition which comprises liposomes and a hydrogel.

In some aspects, the composition is identified for use in inhibiting adsorption of a biofouling-promoting agent on a surface of a substrate (in accordance with any of the respective aspects described herein).

In some aspects, the composition is identified for use in inhibiting biofilm formation on a surface of a substrate (in accordance with any of the respective aspects described herein).

In some aspects described herein, according to any of the aspects described herein, the hydrogel comprises liposomes. In some aspects, the hydrogel comprises substantially all (e.g. at least 90 %, at least 95 %, at least 98 %, at least 99 %) of the liposomes in the composition.

In some aspects described herein, according to any of the aspects described herein, the composition is in a form of a hydrogel comprising the liposomes.

In some aspects described herein, inhibiting adsorption and/or biofilm formation is effected by applying the composition comprising a hydrogel onto a surface of the substrate, thereby contacting the substrate with the composition. Such a substrate may be any type of substrate described herein.

In some aspects described herein, the substrate is not composed of a hydrogel. In some aspects, the substrate does not comprise a hydrogel.

Without being bound by any particular theory, it is believed that substrates which are not composed of a hydrogel, and particularly substrates which do not comprise a hydrogel, are particularly suitable for being treated with a composition comprising a hydrogel and liposomes as described herein, as they cannot be contacted with liposomes by incorporating liposomes within a hydrogel in the substrate, as described herein.

In some aspects described herein, the composition is in a malleable (e.g., in a form of a viscous fluid), which may optionally be applied onto a surface, for example, by being spread onto the surface. In some such aspects, the hydrogel is malleable. In some such aspects, the composition comprises a plurality of hydrogel particles, and the composition is malleable due to the ability of the hydrogel particles to move with respect to one another.

In some aspects described herein, the composition is applied onto a surface by being formed (e.g., in accordance with any of the respective aspects described herein) on the surface. In some such aspects, the composition is not malleable.

In some aspects described herein, a composition comprising a hydrogel is prepared (e.g., as part of a method described herein) by forming the hydrogel in the presence of liposomes.

In some ps described herein, forming a hydrogel comprises, for example, contacting an aqueous liquid with a polymer which forms a hydrogel upon contact with water; and/or forming, in an aqueous liquid, a polymer which forms a hydrogel upon contact with water (e.g., by cross-linking a polymer which does not form a hydrogel prior to cross-linking, to thereby form a cross-linked polymer which forms a hydrogel). In some aspects, the aqueous liquid comprises liposomes, thereby forming a hydrogel in the presence of liposomes.

In some aspects described herein, the liposomes are dispersed throughout the bulk of the hydrogel.

In some aspects described herein, forming the hydrogel is such that the liposomes, and optionally any other component of the hydrogel described herein, are dispersed throughout the bulk of the hydrogel. In some aspects, dispersion throughout the bulk of a hydrogel is effected by cross-linking a polymer in a homogeneous aqueous liquid comprising the liposomes (and optionally other hydrogel components described herein), to thereby form a cross-linked polymer which forms a hydrogel.

Examples of procedures for forming a hydrogel in the presence of liposomes, which may optionally be used to form a hydrogel contacted with liposomes according to any of the respective aspects described herein, are presented in the Examples section herein below, as well as in International Patent Application PCT/IL2014/050604.

In some aspects described herein, the hydrogel is such that inhibition of adsorption (quantified as a percent in reduction of adsorbed agent) is substantially unchanged after a dehydration-rehydration cycle of the hydrogel, that is, dehydration of the hydrogel (e.g., by lyophilization) followed by rehydration of the hydrogel to the water content prior to the dehydration. In some aspects, inhibition of adsorption is unchanged even after more than one dehydration-rehydration cycle of the hydrogel, for example, at least 2, 3, 4, 5, and even 10 cycles.

In some aspects described herein, the hydrogel is such that inhibition of biofilm formation (quantified as a percent in reduction of a biofilm size) is substantially unchanged after a dehydration-rehydration cycle of the hydrogel. In some aspects, inhibition of biofilm formation is unchanged even after more than one dehydration-rehydration cycle of the hydrogel, for example, at least 2, 3, 4, 5, and even 10 cycles.

As used herein, the phrase "substantially unchanged" refers to a value within a range of 50 % to 150 % of an original value. For example, if inhibition of adsorption prior to dehydration-rehydration is 50 % (that is, the inhibition effects a 50 % reduction in adsorption relative to a substrate not contacted with a composition comprising liposomes), and inhibition of adsorption subsequent to dehydration-rehydration is 40 % (that is, the inhibition effects a 40 % reduction in adsorption), the inhibition subsequent to dehydration-rehydration is 80 % of the original inhibition (i.e., 40 % divided by 50 %), and is therefore considered herein to be unchanged.

In some aspects described herein, a unchanged value described herein (e.g., inhibition of adsorption and/or biofilm formation) is in a range of 70 % to 130 % of an original value. In some aspects, a unchanged value is in a range of 80 % to 120 % of an original value. In some aspects, a unchanged value is in a range of 90 % to 110 % of an original value.

### Hydrogel substrate with liposomes:

According to an aspect of the disclosure, there is provided a method of inhibiting adsorption of a biofouling-promoting agent on a surface of a substrate (in accordance with any of the respective aspects described herein) and/or a method of inhibiting biofilm formation on a surface of a substrate (in accordance with any of the respective aspects described herein), the method comprising contacting a substrate composed of a hydrogel with a composition which comprises liposomes.

Herein, the phrase "composed of a hydrogel" refers to a substance (e.g., a substrate described herein) in which at least a portion of the substance consists of a hydrogel. In some of any of the respective aspects, the substance composed of a hydrogel (e.g., a substrate composed of a hydrogel as described herein) primarily consists of a hydrogel, that is, more than 50 % of the volume of the substrate is a hydrogel. In some aspects described herein, the substrate consists of a hydrogel.

In some aspects described herein, at least 50 % of an area of a surface of the substrate (for which adsorption and/or biofilm formation is being inhibited according to any of the aspects described herein) is a surface of a hydrogel. In some aspects, at least 60 % of an area of the surface of the substrate is a surface of a hydrogel. In some aspects, at least 70 % of an area of the surface of the substrate is a surface of a hydrogel. In some aspects, at least 80 % of an area of the surface of the substrate is a surface of a hydrogel. In some aspects, at least 90 % of an area of the surface of the substrate is a surface of a hydrogel. In some aspects, at least 99 % of an area of the surface of the substrate is a surface of a hydrogel.

In some aspects described herein, the contacting of the substrate with the composition comprising liposomes comprises incorporating the composition within the hydrogel. In some such aspects, the composition consists of the liposomes. In some such aspects, the composition consists of the liposomes and a liquid carrier (e.g., water or an aqueous solution).

In some aspects described herein, the substrate is prepared (e.g., as part of a method described herein) by forming a hydrogel which comprises liposomes incorporated in and/or on the hydrogel, thereby contacting the substrate with the liposomes.

In some aspects described herein, the substrate composed of a hydrogel (according to any of the respective aspects described herein) is prepared (e.g., as part of a method described herein) by forming the hydrogel in the presence of liposomes.

In some aspects described herein, forming a hydrogel comprises, for example, contacting an aqueous liquid with a polymer which forms a hydrogel upon contact with water; and/or forming, in an aqueous liquid, a polymer which forms a hydrogel upon contact with water (e.g., by cross-linking a polymer which does not form a hydrogel prior to cross-linking, to thereby form a cross-linked polymer which forms a hydrogel). In some aspects, the aqueous liquid comprises liposomes, thereby forming a hydrogel in the presence of liposomes.

In some aspects described herein, the liposomes are dispersed throughout the bulk of the hydrogel.

In some aspects described herein, forming the hydrogel is such that the liposomes, and optionally any other component of the hydrogel described herein, are dispersed throughout the bulk of the hydrogel. In some aspects, dispersion throughout the bulk of a hydrogel is effected by cross-linking a polymer in a homogeneous aqueous liquid comprising the liposomes (and optionally other hydrogel components described herein), to thereby form a cross-linked polymer which forms a hydrogel.

Examples of procedures for forming a hydrogel in the presence of liposomes, which may optionally be used to form a hydrogel contacted with liposomes according to any of the respective aspects described herein, are presented in the Examples section herein below, as well as in International Patent Application PCT/IL2014/050604.

In some aspects described herein, the substrate is prepared by forming the hydrogel of the substrate in the presence of a solution of polymer (according to any of the respective aspects described herein), optionally in addition to the liposomes. In some aspects, the polymer is not a polymer which forms a hydrogel, but rather an additive, for example, a polymer (e.g., a hydrophilic and/or non-cross-linked polymer described herein) which enhances inhibition of adsorption and/or biofilm formation (e.g., as described herein).

In some aspects described herein, a polymer (which is not a polymer which forms a hydrogel) is dispersed throughout the bulk of the hydrogel. In some aspects, the polymer which forms a hydrogel is intermixed with a polymer (e.g., a cross-linked polymer) which forms the hydrogel.

In some aspects described herein, forming the hydrogel is such that a polymer (which is not a polymer which forms a hydrogel) is dispersed throughout the bulk of the hydrogel. In some aspects, dispersion throughout the bulk of a hydrogel is effected by cross-linking a polymer (which forms a polymer upon cross-linking), to thereby form a cross-linked polymer which forms a hydrogel, in a homogeneous aqueous liquid comprising the polymer which does not form a hydrogel (e.g., a solution of the polymer which does not form a hydrogel), to thereby provide polymer dispersed throughout the bulk of the hydrogel.

### Liposomes and lipids:

The liposomes and/or lipids according to any one of the aspects described in this section may be used in the context of the disclosures described herein.

As used herein and in the art, the term "liposome" refers to an artificially prepared vesicle comprising a bilayer composed of molecules of an amphiphilic lipid. In an aqueous medium, the bilayer is typically configured such that hydrophilic moieties of the amphiphilic lipid are exposed to the medium at both surfaces of the bilayer, whereas lipophilic moieties of the lipid are located in the internal portion of the bilayer, and therefore less exposed to the medium. Examples of liposomes which may be used in any one of the aspects described herein include, without limitation, small unilamellar vesicles, large unilamellar vesicles and multilamellar vesicles.

In some aspects described herein, the liposomes comprise multilamellar vesicles. In some aspects, the liposomes are primarily (more than 50 weight percents) multilamellar vesicles.

In some aspects described herein, the liposomes comprise small unilamellar vesicles. In some aspects, the liposomes are primarily (more than 50 weight percents) small unilamellar vesicles.

In some aspects described herein, the liposomes comprise large unilamellar vesicles. In some aspects, the liposomes are primarily (more than 50 weight percents) large unilamellar vesicles.

As used herein, the term "unilamellar" refers to liposomes characterized by a single lipid bilayer, whereas the term "multilamellar" refers to liposomes characterized by a multiple lipid bilayers, for example, concentric bilayers.

As used herein, the phrase "small unilamellar vesicle" refers to unilamellar liposomes of less than 100 nm in diameter, whereas the phrase "large unilamellar vesicle" refers to unilamellar liposomes at least 100 nm in diameter.

As used herein, the term "amphiphilic lipid" refers to compounds comprising at least one hydrophilic moiety and at least one lipophilic moiety. Examples of amphiphilic lipids include, without limitation, fatty acids (e.g., at least 6 carbon atoms in length) such as phospholipids and glycolipids; sterols (e.g., cholesterol) and steroid acids.

In some aspects described herein, contacting a substrate with the composition comprising liposomes comprises coating the surface with an amphiphilic lipid of the liposomes. Optionally, the surface is coated by a layer of liposomes.

Upon contact with substrate, amphiphilic lipids in a form of liposomes may optionally rearrange in a form other than a liposome, such as a layer (e.g., lipid monolayer and/or lipid bilayer) coating a least a portion of a surface of the substrate.

As used herein, the phrases "amphiphilic lipid of the liposomes", "amphiphilic lipid of said liposomes" refer to any amphiphilic lipid which is present in the liposomes according to any of the respective aspects described herein, and encompasses such lipids even if they are no longer in a liposome (e.g., if the liposome is destroyed).

Herein, the term "phospholipid" refers to a compound comprising a substituted or non-substituted phosphate group and at least one alkyl chain (optionally at least two alkyl chains) which is optionally at least 5 carbon atoms in length, optionally at least 7 atoms in length and optionally at least 9 atoms in length. The at least one alkyl chain is optionally a part of an acyl group (e.g., a fatty acid residue) or an alkyl group *per se* (e.g., a fatty alcohol residue). In some aspects, the phosphate group and on e or two (optionally two) alkyl chains (e.g., acyl or alkyl) are attached to a glycerol moiety via the oxygen atoms of glycerol. The term "phospholipid" encompasses lipids having a (phosphorylated) glycerol backbone (e.g., monoacylglyceride and/or diacylglyceride phospholipids), referred to as glycerophospholipids; and lipids having a (phosphorylated) sphingosine backbone, referred to as phosphosphingolipids (e.g., sphingomyelins).

As used herein, the term "glycolipid" encompasses lipids having a (glycosylated) glycerol backbone (e.g., monoacylglyceride and/or diacylglyceride glycolipids), referred to as glyceroglycolipids; and lipids having a (glycosylated) sphingosine backbone, referred to as glycosphingolipids (e.g., cerebrosides, gangliosides).

In some aspects described herein, the amphiphilic lipid comprises a hydrophilic moiety which is an ionic moiety.

Herein, the phrase "ionic moiety" refers to a moiety which comprises at least one charged group (as defined herein), and includes anionic moieties (which have a net negative charge), cationic moieties (which have a net positive charge) and zwitterionic moieties (which have an equal number of positive and negative charges, and thus, no net charge).

Without being bound by any particular theory, it is believed that ionic moieties are particularly effective at binding to water molecules, which renders lipid molecules comprising such moieties particularly effective at promoting hydration layers near the surface which can serve as a barrier to adsorption onto the surface.

In some aspects described herein, the amphiphilic lipid of the liposomes comprises at least one phospholipid. Phospholipids are typically characterized by the presence of an ionic moiety which includes a negative charge associated with an oxygen atom in a phosphate moiety (P-O⁻), although additional charges may be present.

In some aspects described herein, the phospholipid is a glycerophospholipid. In some aspects, the glycerophospholipid is a diacylglyceride, comprising two fatty acyl groups and one phosphate group attached to a glycerol backbone.

In some aspects described herein, a concentration of phospholipids in liposomes in a composition described herein (e.g., a solution described herein) is in a range of from 0.1 mM to 500 mM. In some aspects, the concentration is in a range of from 0.3 mM to 150 mM. In some aspects, the concentration is in a range of from 1 mM to 50 mM. In some aspects, the concentration is in a range of from 2 mM to 15 mM.

In some aspects described herein, a concentration of phospholipids in liposomes in a composition described herein (e.g., a solution described herein) is in a range of from 0.1 mM to 50 mM. In some aspects, the concentration is in a range of from 0.3 mM to 15 mM.

In some aspects described herein, a concentration of phospholipids in liposomes in a composition described herein (e.g., a solution described herein) is in a range of from 0.3 mM to 500 mM. In some aspects, the concentration is in a range of from 1 mM to 150 mM. In some aspects, the concentration is in a range of from 2 mM to 50 mM.

In some aspects described herein, the amphiphilic lipid of the liposomes comprises at least one negatively charged atom and at least one positively charged atom. In some aspects, the amphiphilic lipid is zwitterionic, that is, the one or more negative charges in the molecule are balanced out by an equal number of positive charge(s) in the molecule. In some aspects, the amphiphilic lipid comprises exactly one negative charge and one positive charge.

In some aspects described herein, the amphiphilic lipid of the liposomes comprises at least one phospholipid which comprises a phosphoethanolamine group or N-alkyl derivative thereof. In some aspects, the phospholipid is a glycerophospholipid.

The phrase "phosphoethanolamine group or N-alkyl derivative thereof' refers to a -O-P(=O)(-O⁻)-OCH₂CH₂NR'R"R'"⁺ group (or a salt thereof), wherein R', R" and R'" are each independently hydrogen or alkyl, preferably C₁₋₄ alkyl. In some aspects described herein, the alkyl groups attached to the nitrogen atom are each independently methyl or ethyl. In some aspects, the alkyl(s) is methyl. The term "phosphoethanolamine" refers to a group wherein R', R" and R'" are each hydrogen. The term "phosphocholine" refers to a group wherein R', R" and R'" are each methyl.

Without being bound by any particular theory, it is believed that the distance between the positive and negative charges in a phosphoethanolamine group or N-alkyl derivative thereof is particularly suitable for binding water molecules and/or promoting hydration.

In some aspects described herein, a molar percentage of the phospholipid described herein (e.g., in liposomes described herein) which comprises a phosphoethanolamine group or N-alkyl derivative thereof is at least 20 %. In some aspects, the molar percentage is at least 40 %. In some aspects, the molar percentage is at least 50 %. In some aspects, the molar percentage is at least 60 %. In some aspects, the molar percentage is at least 70 %. In some aspects, the molar percentage is at least 80 %. In some aspects, the molar percentage is at least 90 %. In some aspects, the phospholipid consists of at least one phospholipid comprising a phosphoethanolamine group or N-alkyl derivative thereof.

In some aspects described herein, a molar percentage of the amphiphilic lipid of the liposomes described herein which consists of at least one phospholipid which comprises a phosphoethanolamine group or N-alkyl derivative thereof is at least 20 %. In some aspects, the molar percentage is at least 40 %. In some aspects, the molar percentage is at least 50 %. In some aspects, the molar percentage is at least 60 %. In some aspects, the molar percentage is at least 70 %. In some aspects, the molar percentage is at least 80 %. In some aspects, the molar percentage is at least 90 %. In some aspects, the amphiphilic lipid consists of at least one phospholipid which comprises a phosphoethanolamine group or N-alkyl derivative thereof.

In some aspects described herein, the at least one phospholipid comprises at least one phosphatidylcholine.

Herein and in the art, the term "phosphatidylcholine" refers to a glycerophospholipid comprising a phosphocholine group and two fatty acyl groups attached to a glycerol backbone (i.e., a diacylglyceride).

In some aspects described herein, the phospholipid described herein (e.g., in liposomes described herein) is characterized by a molar percentage of phosphatidylcholine (the at least one phosphatidylcholine described herein) which is at least 20 %. In some aspects, the molar percentage is at least 40 %. In some aspects, the molar percentage is at least 50 %. In some aspects, the molar percentage is at least 60 %. In some aspects, the molar percentage is at least 70 %. In some aspects, the molar percentage is at least 80 %. In some aspects, the molar percentage is at least 90 %. In some aspects, the phospholipid consists of at least one phosphatidylcholine.

In some aspects described herein, the amphiphilic lipid of the liposomes described herein is characterized by a molar percentage of phosphatidylcholine (the at least one phosphatidylcholine described herein) which is at least 20 %. In some aspects, the molar percentage is at least 40 %. In some aspects, the molar percentage is at least 50 %. In some aspects, the molar percentage is at least 60 %. In some aspects, the molar percentage is at least 70 %. In some aspects, the molar percentage is at least 80 %. In some aspects, the molar percentage is at least 90 %. In some aspects, the amphiphilic lipid consists of at least one phosphatidylcholine.

The fatty acyl groups in a lipid described herein may comprise saturated fatty acyl groups, monounsaturated fatty acyl groups (having a single unsaturated bond) and/or polyunsaturated fatty acyl groups (having two or more unsaturated bonds). In some aspects, the unsaturated bonds are *cis* double bonds.

Examples of suitable saturated fatty acyl groups include, without limitation, lauroyl, myristoyl, palmitoyl and stearoyl.

Examples of suitable monounsaturated fatty acyl groups include, without limitation, oleoyl, palmitoleoyl, eicosenoyl, erucoyl, nervonoyl and vaccenoyl.

Examples of suitable polyunsaturated fatty acyl groups include, without limitation, linoleoyl, α-linolenoyl, γ-linolenoyl, dihomo-γ-linolenoyl, stearidonoyl, eicosatetraenoyl, eicosapentaenoyl, docosapentaenoyl, docosahexaenoyl, arachidonoyl and adrenoyl.

In some aspects described herein, the fatty acyl groups are selected from the group consisting of saturated and monounsaturated fatty acyl groups. In some aspects, the fatty acyl groups are saturated fatty acyl groups.

Without being bound by any particular theory, it is believed that saturated and monounsaturated fatty acyl groups, particularly saturated fatty acyl groups, are relatively resistant to chemical reaction such as oxidation, and therefore provide a more resilient system.

In some aspects described herein, at least 50 % of the fatty acyl groups are the same species of fatty acyl group (e.g., myristoyl, palmitoyl). In some aspects, at least 75 % of the fatty acyl groups are the same species of fatty acyl group. In some aspects, at least 90 % of the fatty acyl groups are the same species of fatty acyl group.

1,2-dimyristoyl-sn-glycero-3-phosphocholine is an example of a phospholipid comprising a single species of fatty acyl group.

It is to be appreciated that phase transitions, e.g., melting points (Tm), of the lipid bilayers and liposomes described herein may be determined by the skilled person by selecting suitable fatty acyl groups for inclusion in the lipids, for example, by selecting relatively short and/or unsaturated fatty acyl groups (e.g., myristoyl) to obtain a relatively low melting point; and/or by selecting relatively long and/or saturated fatty acyl groups (e.g., palmitoyl and/or stearoyl) to obtain a relatively high melting point.

In some aspects described herein, the liposomes described herein are characterized by a phase transition melting point below an expected ambient temperature of a surface to which the liposomes are applied (e.g., as described herein in any one of the respective aspects), such that a surface coated by lipids at the expected ambient temperature (e.g., about 36 °C on a contact lens surface in contact with an eye, about 20 °C or 25 °C in a non-physiological environment) will be coated predominantly by lipids in a liquid phase.

In some aspects described herein, the liposomes are characterized by a phase transition melting point (Tm) below 37 °C. In some aspects, the Tm is below 30 °C. In some aspects, the Tm is below 25 °C. In some aspects, the Tm is below 20 °C.

1,2-dimyristoyl-sn-glycero-3-phosphocholine (Tm = 24 °C) is an example of a phospholipid characterized by a Tm below 25 °C. Thus, liposomes consisting of such a phospholipid will be characterized by a Tm of 24 °C. Determination of a Tm of liposomes containing 1,2-dimyristoyl-sn-glycero-3-phosphocholine in combination with one or more other lipids is well withing the capabilities of one of skill in the art.

In some aspects relating to a method described herein, the liposomes described herein are characterized by a phase transition melting point below a temperature of a surface (e.g., a temperature described herein) during contacting of the liposomes with the surface (according to any of the respective aspects described herein).

In some aspects described herein, liquid phase liposomes exhibit a superior anti-biofouling effect as compared to solid phase liposomes.

Lipid coatings in a liquid phase have been reported previously to provide the most effective hydration and/or lubrication.

However, in some aspects described herein wherein liquid phase liposomes are superior to solid phase liposomes in providing an anti-biofouling effect, such superiority is considerably greater than the differences in hydration and/or lubrication efficacy between liquid phase and solid phase liposomes, for example, due to a superior ability of liquid phase liposomes to coat a surface with a lipid with minimal gaps in a lipid layer on a surface.

In some aspects described herein wherein the liposomes are characterized by a relatively high phase transition melting point (Tm) (e.g., at least 37 °C, at least 30 °C, at least 25 °C) and/or wherein the liposomes described herein are characterized by a phase transition melting point above a temperature of a surface during contacting of the liposomes with the surface (according to any of the respective aspects described herein), the liposomes are used in combination with a polymer according to any of the respective aspects described herein. In some such aspects, the polymer comprises hydrophobic regions therein (e.g., as described herein).

In some aspects described herein, the liposomes described herein are characterized by a surface charge, which may be a positive surface charge or a negative surface charge.

As used herein, the phrase "surface charge" refers to an electric charge at or near a surface, such as an interface of a liposome with a solution. The phrase "surface charge" encompasses an electric charge associated with an electric potential at a surface (e.g., such that a positive electric potential at a surface is indicative of a positive surface charge, whereas a negative electric potential at a surface is indicative of a negative surface charge); as well as an electric charge which is closer to a surface than an electric charge of an opposite sign (e.g., as in a zwitterion wherein the positive charge is closer to the surface than the negative charge, or *vice versa*), such that an ion near the surface will interact primarily with the electric charge near the surface (due to the proximity) as opposed to the electric charge of an opposite sign. For example, phosphatidylcholine liposomes typically exhibit a positive surface charge because the positive charge of the choline group is closer to the liposome surface than the negative charge of the phosphate group.

Optionally, a surface charge of a liposome is associated with a net charge of the lipid molecules in the liposome, for example, a liposome comprising anionic lipids has a negative surface charge, and/or a liposome comprising cationic lipids has a positive surface charge.

Alternatively or additionally, a surface charge of a liposome is associated with a dipole of lipid molecules (e.g., zwitterionic lipid molecules) in the liposome, for example, a liposome comprising a zwitterionic lipid comprising a phosphocholine group may have a positive surface charge due to the positively charged ammonium groups in the phosphocholine groups being (on average) closer to the surface of the liposomes than the negatively charged phosphate groups in the phosphocholine groups.

The skilled person will be readily capable of determining a surface charge. For example, the sign of a surface charge may be determined by comparing the propensity of a surface (e.g., of a liposome) to bind to anionic vs. cationic compounds (e.g., labeling compounds).

In some aspects described herein, the liposomes rupture upon contact with the substrate (e.g., in aspects in which the liposomes are contacted with a surface of the substrate). Liposome rupture may optionally result in a lipid bilayer in the liposomes being converted from a curved geometry (e.g., as in the relatively spherical liposomes) to a flatter geometry which complements the geometry of the substrate surface and/or molecules attached to the surface (e.g., thereby enhancing affinity of the lipids to the surface), thereby facilitating coating of the surface with a lipid (e.g., as described herein); and/or which results in a flatter, smoother lipid-coated surface, which may further inhibit adsorption and/or biofilm formation on the surface.

Without being bound by any particular theory, it is believed that rupture of liposomes may be induced by affinity of the polymer described herein to the lipids in the liposome, whereby rupture of the liposomes allows a greater area of the polymer to come into contact with lipids, thereby increasing an amount of energetically favorable interactions between the polymer and lipid.

In some aspects described herein, liposomes and polymer are selected such that the selected polymer is effective at rupturing the selected liposomes.

### Polymer:

In some aspects of the disclosure described herein, a polymer is utilized in combination with the liposomes described herein.

Herein throughout, the term "polymer" refers to a polymer utilized in combination with the liposomes (as opposed, for example, to a component of a substrate and/or hydrogel described herein), unless indicated otherwise.

The polymer according to any one of the aspects described in this section may be used in the context of the disclosures described herein, and in combination with liposomes according to any one of the aspects described herein with respect to liposomes.

In some aspects described herein relating to a method, the method further comprises contacting the substrate with the polymer.

In some aspects described herein, contacting the substrate with the polymer is effected prior to contacting the substrate with the composition comprising liposomes.

In some aspects described herein, contacting the substrate with the polymer is effected subsequent to contacting the substrate with the composition comprising liposomes.

In some aspects described herein, contacting the substrate with the polymer is effected concomitantly with contacting the substrate with the composition comprising liposomes.

In some aspects described herein, according to any of the aspects described herein, the composition comprising liposomes further comprises the polymer. Such a composition may optionally be used to effect concomitant contacting of the substrate with the composition and polymer.

In some aspects described herein, the composition comprising the polymer and liposomes is a liquid (e.g., as described herein) and the polymer is dissolved and/or dispersed in the liquid. In some aspects, the polymer is dissolved in the liquid.

In some aspects described herein, the composition comprising the polymer and liposomes comprises a hydrogel (e.g., as described herein) and the polymer is dispersed throughout the bulk of the hydrogel.

In some aspects described herein relating to a composition comprising a hydrogel, the composition is prepared by forming the hydrogel in the presence of a solution of the polymer. In some aspects, the forming of the hydrogel is such that the polymer is dispersed throughout the bulk of the hydrogel.

In some aspects described herein, the polymer utilized in combination with the liposomes is not a polymer which forms a hydrogel described herein.

In some aspects described herein, the polymer is a non-cross-linked polymer. In some such aspects, the hydrogel is formed from a cross-linked polymer, and the non-cross-linked polymer is thus different than the polymer which forms a hydrogel.

Herein, the phrase "non-cross-linked polymer" encompasses polymers in which a degree of cross-linking (e.g., covalent cross-linking) is sufficiently low such that the molecules of the polymer (which optionally comprise a plurality of cross-linked polymer chains) have an average molecular weight (weight-averaged average molecular weight) which is not more than 10 MDa (10,000,000 Da).

In some aspects described herein, an average molecular weight (weight-averaged average molecular weight) of the polymer is 3 MDa (3,000,000 Da) or less. In some aspects, the average molecular weight of the polymer is 1 MDa or less. In some aspects, the average molecular weight of the polymer is 300 kDa or less. In some aspects, the average molecular weight of the polymer is 100 kDa or less. In some aspects, the average molecular weight of the polymer is 30 kDa or less.

In some aspects described herein, the polymer is a hydrophilic polymer.

Herein, the phrase "hydrophilic polymer" encompasses polymers which are water-soluble and/or water-swellable.

In some aspects described herein, the hydrophilic polymer is water-soluble.

In some aspects described herein, the hydrophilic polymer is water-swellable.

Herein, the term "water-soluble" refers to a solubility of at least 1 gram per liter in water at a pH of 7.

In some aspects described herein, a water-soluble polymer has a solubility of at least 3 grams per liter in water at a pH of 7. In some aspects, the solubility is at least 10 grams per liter. In some aspects, the solubility is at least 30 grams per liter. In some aspects, the solubility is at least 100 grams per liter.

Herein, the term "water-swellable" refers to an ability of a solid substance to absorb at least 10 weight percents water (weight of water per weight of solid) upon contact with water at a pH of 7.

In some aspects described herein, a water-swellable polymer has an ability to absorb at least 30 weight percents water (weight of water per weight of solid) upon contact with water at a pH of 7. In some aspects, the water-swellable polymer has an ability to absorb at least 20 weight percents water. In some aspects, the water-swellable polymer has an ability to absorb at least 50 weight percents water. In some aspects, the water-swellable polymer has an ability to absorb at least 100 weight percents water. In some aspects, the water-swellable polymer has an ability to absorb at least 200 weight percents water (twice the weight of the polymer *per se*).

Examples of hydrophilic polymers include, without limitation, polypeptides, such as gelatin; non-ionic hydrophilic polymers, such as poly(2-hydroxyethyl methacrylate), hydroxypropyl methyl cellulose (HPMC), hydroxypropyl cellulose (HPC), methyl cellulose, dextran, polyvinylpyrrolidone (PVP), polyethylene glycol (also referred to herein interchangeably as "polyethylene oxide" or "PEO") and polyvinyl alcohol; ionic polymers, such as polymethacrylic acid, carboxymethyl cellulose, hyaluronic acid, chondroitin sulfate, alginic acid, xanthan gum, chitosan and N-alkyl chitosan; and copolymers thereof. Poly(2-hydroxyethyl methacrylate), polyvinylpyrrolidone, polyethylene oxide and hydroxypropyl methyl cellulose are exemplary non-ionic hydrophilic polymers.

In some aspects described herein relating to a polymer, the polymer is a non-ionic polymer, for example, a polymer selected from the group consisting of polyvinylpyrrolidone, polyethylene oxide and hydroxypropyl methyl cellulose.

In some aspects described herein relating to a polymer, the polymer is hydroxypropyl methyl cellulose. As exemplified herein, both solid phase and liquid phase liposomes in combination with hydroxypropyl methyl cellulose are effective at reducing adhesion.

In some aspects described herein, the polymer comprises hydrophobic regions (e.g., in aspects wherein the liposomes have a relatively high melting point and/or have a melting point above a temperature of a surface to which they are contacted, as described herein).

Examples of polymers (e.g., hydrophilic polymers) comprising hydrophobic regions include, without limitation, cellulose (e.g., HPMC, HPC, methyl cellulose, ethyl cellulose, carboxymethyl cellulose), poly(vinyl methyl ether), polyethacrylic acid and esters (e.g., methyl esters) thereof, polymethacrylate and/or polyacrylate esters (e.g., poly(2-hydroxyethyl methacrylate).

As used herein, the phrase "ionic polymer" refers to a polymer is characterized by a charge density of at least 1 charged group (ionic group) per 1 kDa molecular weight of the polymer in an aqueous (e.g., water) environment at pH 7. The phrase "ionic polymer" encompasses polymers having a net negative charge (also referred to herein as "anionic polymers"), polymers having a net positive charge (also referred to herein as "cationic polymers"), and polymers having no net charge (also referred to herein as "zwitterionic polymers"), in an aqueous (e.g., water) environment at pH 7.

As used herein, the phrase "non-ionic hydrophilic polymer" encompasses any hydrophilic polymer (as defined herein) which is not an ionic polymer (as defined herein).

Herein throughout, the phrase "charged group" refers to any functional group (e.g., a functional group described herein) which is ionic (as defined herein), including, for example, amine, carboxylic acid, sulfate, sulfonate, phosphate and phosphonate. Thus, each electric charge in a moiety or molecule is associated with one charged group, although a single charged group (e.g., non-substituted phosphate) may be associated with more than one electric charge of the same sign (e.g., a dianion, a dication).

In some aspects described herein, at least 75 % of the ionic groups in the ionic polymer have the same charge, that is, at least 75 % of the ionic groups are cationic groups or are anionic groups, such that the ionic polymer is cationic or anionic, respectively. In some aspects, at least 90 % of the ionic groups in the ionic polymer have the same charge. In some aspects, at least 95 % of the ionic groups in the ionic polymer have the same charge. In some aspects, at least 98 % of the ionic groups in the ionic polymer have the same charge. In some aspects, at least 99 % of the ionic groups in the ionic polymer have the same charge.

In some aspects described herein, about 50 % of the ionic groups in the ionic polymer have a positive charge and about 50 % of the ionic groups in the ionic polymer have a negative charge, such that the ionic polymer is zwitterionic.

In some aspects described herein, the ionic polymer has from 1 to 6 charged groups per 1 kDa. In some aspects, the ionic polymer has from 1.5 to 4 charged groups per 1 kDa. In some aspects, the ionic polymer has from 2 to 3 charged groups per 1 kDa.

In some aspects described herein, the ionic polymer is characterized by a net charge (i.e., the difference between the number of anionic groups and the number of cationic groups) of from 1 to 6 electric charges per 1 kDa molecular weight of the polymer. In some aspects, the ionic polymer has a net charge of from 1.5 to 4 charges per 1 kDa. In some aspects, the ionic polymer has a net charge of from 2 to 3 charges per 1 kDa.

In some aspects described herein, the ionic polymer is an anionic polymer, for example, a polymer characterized by a net negative charge of from 1 to 6 electric charges per 1 kDa molecular weight of the polymer.

In some aspects described herein, the ionic polymer is a cationic polymer, for example, a polymer characterized by a net positive charge of from 1 to 6 electric charges per 1 kDa molecular weight of the polymer.

In some aspects described herein, the polymer is a polysaccharide. In some such aspects, the polysaccharide is an ionic polymer. In some such aspects, the polysaccharide is a non-ionic polymer.

As used herein, the term "polysaccharide" refers to a polymer composed primarily (at least 50 weight percents) of monosaccharide units linked by glycosidic linkages.

As used herein, the term "monosaccharide" encompasses carbohydrates *per se* (having the formula Cn(H₂O)n, wherein n is at least 3, typically from 3 to 10), such as amino sugars, in which at least one hydroxyl group is replaced by an amine or amide group; sugar acids, in which one or two carbon atoms are oxidized to form a carboxylate group; acylated monosaccharides, in which at least one hydroxyl group and/or amine group is substituted by an acyl group (e.g., acetyl); monosaccharide ethers (e.g., cellulose ethers), in which at least one hydroxyl group is substituted by an alkyl group (e.g., methyl, carboxymethyl, hydroxypropyl); and sulfated monosaccharides, in which at least one hydroxyl group is replaced by a sulfate group.

Examples of monosaccharides include, without limitation, hexoses (e.g., D-hexoses and/or L-hexoses) such as allose, altrose, glucose, mannose, gulose, idose, galactose, talose, psicose, fructose, sorbose and tagatose; pentoses (e.g., D-pentoses and/or L-pentoses) such as arabinose, lyxose, xylose, ribose, ribulose and xylulose; and hexose such as glucuronic acid, iduronic acid, mannuronic acid, guluronic acid, glucosamine and N-alkyl , galactosamine and N-alkyl, N-acetylglucosamine, N-acetylgalactosamine, and monosulfated and disulfated N-acetylgalactosamine, glucuronic acid and iduronic acid.

As used herein, the phrase "glycosidic linkage" refers to a bond between a hemiacetal group of one compound (e.g., a monosaccharide monomer) and a hydroxyl group of another compound (e.g., another monosaccharide monomer).

Examples of polysaccharides which are non-ionic polymers include, without limitation, dextran and cellulose such as hydroxypropyl methyl cellulose, hydroxypropyl cellulose, and methyl cellulose.

Examples of polysaccharides which are ionic polymers include, without limitation, carboxymethyl cellulose, hyaluronic acid, chondroitin sulfate, alginic acid, xanthan gum, chitosan and N-alkyl chitosan.

Hyaluronic acid is an anionic polysaccharide comprising anionic glucuronic acid monomer units along with non-ionic N-acetylglucosamine monomer units. Hyaluronic acid is an exemplary anionic polymer.

Chondroitin sulfate is an anionic polysaccharide comprising anionic sulfated (e.g., monosulfated and/or disulfated) N-acetylgalactosamine, glucuronic acid and/or iduronic acid monomer units, and anionic glucuronic acid and/or iduronic acid monomer units, along with non-ionic N-acetylgalactosamine monomer units.

Alginic acid is an anionic polysaccharide comprising anionic mannuronic acid and guluronic acid monomer units.

Xanthan gum is an anionic polysaccharide comprising anionic glucuronic acid monomer units, along with non-ionic glucose and mannose monomer units (including acetyl and/or pyruvyl).

Chitosan is a cationic polysaccharide comprising cationic glucosamine monomer units, optionally along with non-ionic N-acetylglucosamine monomer units. In N-alkyl chitosan, at least a portion of the glucosamine units comprise 1, 2 or 3 alkyl groups, preferably C₁₋₄ alkyl, attached to the nitrogen atom. In some aspects described herein, the alkyl groups attached to the nitrogen atoms are each independently methyl or ethyl. In some aspects, the alkyls are methyl. In some aspects, the N-alkylated monomer unit is N-trimethylglucosamine.

Herein, the terms "hyaluronic acid", "chondroitin sulfate", "alginic acid", "xanthan gum", "chitosan", "N-alkyl chitosan" and any other ionic compounds named herein, encompass all salts of the named compounds along with the non-ionic forms (e.g., acid forms of the anionic polysaccharides, and the free base forms of the cationic polysaccharides).

In some aspects described herein, the ionic polymer (e.g., ionic polysaccharide) is in a form of a salt. In some aspects, the salt is a pharmaceutically acceptable salt (e.g., an ophthalmically acceptable salt for an ophthalmic application described herein).

In some aspects described herein relating to an ionic polymer which is a polysaccharide, the polysaccharide has from 0.2 to 1 charged groups per monosaccharide residue. In some aspects, the polysaccharide has from 0.2 to 0.9 charged groups per monosaccharide residue. In some aspects, the polysaccharide has from 0.3 to 0.7 charged groups per monosaccharide residue. In some aspects, the polysaccharide has from 0.4 to 0.6 charged groups per monosaccharide residue. In some aspects, the polysaccharide has about 0.5 charged groups per monosaccharide residue.

It is to be appreciated that a monosaccharide residue may comprise more than one charged group (e.g., a sulfate group and a carboxylate group).

In some aspects described herein relating to an ionic polymer which is a polysaccharide, the monosaccharide residues comprise no more than one charged group, that is, 0 or 1 charged group.

In some aspects described herein relating to an ionic polymer which is a polysaccharide, the polysaccharide is characterized by a net charge (i.e., the difference between the number of anionic groups and the number of cationic groups) of from 0.2 to 1 electric charges per monosaccharide residue. In some aspects, the net charge is from 0.2 to 0.9 electric charges per monosaccharide residue. In some aspects, the net charge is from 0.3 to 0.7 electric charges per monosaccharide residue. In some aspects, the net charge is from 0.4 to 0.6 electric charges per monosaccharide residue. In some aspects, the net charge is about 0.5 electric charges per monosaccharide residue.

In some aspects described herein, a molecular weight (e.g., weight averaged molecular weight) of the polymer is in a range of from 5 kDa to 10 MDa. In some aspects, a molecular weight is from 10 kDa to 5 MDa. In some aspects, a molecular weight is from 20 kDa to 2 MDa. In some aspects, a molecular weight is from 50 kDa to 1 MDa.

In some aspects described herein, the molecular weight (weight averaged molecular weight) is from 5 kDa to 5 MDa. In some aspects, a molecular weight is from 5 kDa to 2 MDa. In some aspects, a molecular weight is from 5 kDa to 1 MDa. In some aspects, a molecular weight is from 5 kDa to 500 kDa. In some aspects, a molecular weight is from 5 kDa to 200 kDa. In some aspects, a molecular weight is from 5 kDa to 100 kDa.

In some aspects described herein, the molecular weight (weight averaged molecular weight) is from 10 kDa to 10 MDa. In some aspects, a molecular weight is from 20 kDa to 10 MDa. In some aspects, a molecular weight is from 50 kDa to 10 MDa. In some aspects, a molecular weight is from 100 kDa to 10 MDa. In some aspects, a molecular weight is from 200 kDa to 10 MDa. In some aspects, a molecular weight is from 500 kDa to 10 MDa.

In some aspects described herein, the polymer is selected to enhance an affinity of the liposomes to a surface of the substrate, that is, the liposome lipids have a greater affinity to the surface contacted with (e.g., coated by) the polymer than to the surface in the absence of the polymer.

In some aspects described herein relating to an ionic polymer, the ionic polymer is selected such that the liposomes are characterized by a surface charge having a sign opposite a sign of a net charge of the ionic polymer.

In some aspects described herein, the liposomes are characterized by a negative surface charge (e.g., as described herein in any one of the respective aspects) and the ionic polymer has a net positive charge (e.g., as described herein in any one of the respective aspects). In some aspects, the ionic polymer is a polysaccharide having a net positive charge (e.g., a cationic polysaccharide described herein in any one of the respective aspects).

In some aspects described herein, the liposomes are characterized by a positive surface charge (e.g., as described herein in any one of the respective aspects) and the ionic polymer has a net negative charge (e.g., as described herein in any one of the respective aspects). In some aspects, the ionic polymer is a polysaccharide having a net negative charge (e.g., an anionic polysaccharide described herein in any one of the respective aspects). In some aspects, the ionic polymer is hyaluronic acid or alginic acid (optionally hyaluronate or alginate salts, in accordance with the definitions of "hyaluronic acid" and "alginic acid" used herein).

In some aspects described herein, the amphiphilic lipid of the liposomes comprises at least one phospholipid which comprises a phosphoethanolamine group or N-alkyl derivative thereof (e.g., in any one of the respective aspects) and the ionic polymer has a net negative charge (e.g., as described herein in any one of the respective aspects). In some aspects, the lipid is a phosphatidylcholine (e.g., as described herein in any one of the respective aspects). In some aspects, the ionic polymer is a polysaccharide having a net negative charge (e.g., an anionic polysaccharide described herein). In some aspects, the ionic polymer is hyaluronic acid or alginic acid.

In some aspects described herein, a concentration of polymer in a composition described herein is in a range of from 0.01 to 100 mg/ml. In some aspects, the concentration is in a range of from 0.03 to 100 mg/ml. In some aspects, the concentration is in a range of from 0.1 to 100 mg/ml. In some aspects, the concentration is in a range of from 0.3 to 100 mg/ml. In some aspects, the concentration is in a range of from 1 to 100 mg/ml. In some aspects, the concentration is in a range of from 3 to 100 mg/ml. In some aspects, the concentration is in a range of from 10 to 100 mg/ml.

In some aspects described herein, a concentration of polymer in a composition described herein is in a range of from 0.01 to 30 mg/ml. In some aspects, the concentration is in a range of from 0.03 to 30 mg/ml. In some aspects, the concentration is in a range of from 0.1 to 30 mg/ml. In some aspects, the concentration is in a range of from 0.3 to 30 mg/ml. In some aspects, the concentration is in a range of from 1 to 30 mg/ml. In some aspects, the concentration is in a range of from 3 to 30 mg/ml.

In some aspects described herein, a concentration of polymer in a composition described herein is in a range of from 0.01 to 10 mg/ml. In some aspects, the concentration is in a range of from 0.03 to 10 mg/ml. In some aspects, the concentration is in a range of from 0.1 to 10 mg/ml. In some aspects, the concentration is in a range of from 0.3 to 10 mg/ml. In some aspects, the concentration is in a range of from 1 to 10 mg/ml.

In some aspects described herein, a concentration of polymer in a composition described herein is less than 3 mg/ml. In some aspects, the concentration is at least 0.01 mg/ml. In some aspects, the concentration is at least 0.03 mg/ml. In some aspects, the concentration is at least 0.1 mg/ml. In some aspects, the concentration is at least 0.3 mg/ml.

In some aspects described herein, a concentration of polymer in a composition described herein is less than 1 mg/ml. In some aspects, the concentration is at least 0.01 mg/ml. In some aspects, the concentration is at least 0.03 mg/ml. In some aspects, the concentration is at least 0.1 mg/ml. In some aspects, the concentration is at least 0.3 mg/ml.

In some aspects described herein, a concentration of polymer in a composition described herein is less than 0.5 mg/ml. In some aspects, the concentration is at least 0.01 mg/ml. In some aspects, the concentration is at least 0.03 mg/ml. In some aspects, the concentration is at least 0.1 mg/ml.

Herein, for any one of the aspects and for any combination thereof, a composition which comprises liposomes (optionally combined with a hydrogel and/or a polymer) is to be regarded as "anti-biofouling (ABF) composition".

### Contact lenses and keratitis:

According to another aspect of the disclosure, there is provided a method of inhibiting biofilm formation on a contact lens, the method comprising rinsing and/or immersing the contact lens in a solution comprising liposomes and an aqueous carrier. The method is optionally in accordance with a method of inhibiting biofilm formation on a surface of a substrate (according to any of the respective aspects described herein), wherein the substrate is a contact lens.

In some aspects described herein, the method is for inhibiting formation of a biofilm of a pathogenic microorganism associated with keratitis.

According to another aspect of the disclosure, there is provided a method of treating keratitis in a contact-lens-wearing subject in need thereof, the method comprising rinsing and/or immersing a contact lens in a solution comprising liposomes and an aqueous carrier (according to any of the respective aspects described herein), prior to insertion of the contact lens in an eye of the subject.

According to another aspect of the disclosure, there is provided a use of a solution comprising liposomes and an aqueous carrier in the manufacture of a medicament for the treatment of keratitis in a contact-lens-wearing subject in need thereof, the treatment comprising rinsing and/or immersing a contact lens in a solution comprising liposomes and an aqueous carrier (according to any of the respective aspects described herein), prior to insertion of the contact lens in an eye of the subject.

According to another aspect of the disclosure, there is provided a solution comprising liposomes and an aqueous carrier (according to any of the respective aspects described herein), for use in the treatment of keratitis in a contact-lens-wearing subject in need thereof, the treatment comprising rinsing and/or immersing a contact lens in a solution comprising liposomes and an aqueous carrier, prior to insertion of the contact lens in an eye of the subject.

Without being bound by any particular theory, it is believed that a solution which inhibits biofilm formation (e.g., according to any of the respective aspects described herein) is useful in treating keratitis even if the solution has no direct effect on the responsible pathogen at the site of infection in the eye, as the inhibition of biofilm formation on the contact lens reduces the likelihood and/or degree of re-infection, thereby facilitating recovery, and/or reduces the risk of initiation of keratitis (e.g., as a prophylactic treatment). It is further believed that the pathogen at the site of infection may be removed by flow of tear fluid, an immune response, and or by therapy, for example, by action of a drug (e.g., administration of a suitable antimicrobial agent), and that the therapeutic efficacy of such removal of the pathogen is enhanced by inhibition of biofilm formation on the contact lens.

In some aspects described herein relating to keratitis, according to any of the aspects described herein, the keratitis is associated with contact lens use (e.g., diagnosed by a physician as being associated with contact lens use, for example, based on the symptoms of a contact lens user and/or on the infecting pathogen).

Examples of pathogenic microorganisms associated with keratitis (as well as keratitis which is associated with contact lenses) include, without limitation, amoebas (e.g., *Acanthamoeba*), bacteria (e.g., *Staphylococcus aureus, Pseudomonas aeruginosa*) and fungi (e.g., *Fusarium*).

In some aspects described herein, according to any of the aspects described herein, the solution further comprises at least one polymer. In some aspects, the polymer is a hydrophilic polymer. In some aspects, the hydrophilic polymer is a water-soluble polymer. In some aspects, the water-soluble polymer is entirely dissolved, that is, the concentration of polymer is at or below the saturation point.

Without being bound by any particular theory, it is believed that inclusion of small unilamellar vesicles and/or dissolved polymer (as opposed to another form of liposome and/or polymer) allows for minimal effects on the transparency of the contact lens, due to the relatively low light scattering by small unilamellar vesicles and/or dissolved polymer molecules in comparison with larger particles.

In some aspects described herein relating to a contact lens and/or keratitis, the liposomes are characterized by a phase transition melting point (Tm) below 37 °C. In some aspects, the Tm is below 36 °C. In some aspects, the Tm is below 35 °C. In some aspects, the Tm is below 34 °C. In some aspects, the Tm is below 32 °C. In some aspects, the Tm is below 30 °C. In some aspects, the Tm is below 25 °C. In some aspects, the Tm is below 20 °C.

In some aspects described herein, a concentration of polymer in the solution is in a range of from 0.01 to 10 mg/ml. In some aspects, the concentration is in a range of from 0.03 to 10 mg/ml. In some aspects, the concentration is in a range of from 0.1 to 10 mg/ml. In some aspects, the concentration is in a range of from 0.3 to 10 mg/ml.

In some aspects described herein, a concentration of polymer in the solution is less than 3 mg/ml. In some aspects, the concentration is at least 0.01 mg/ml. In some aspects, the concentration is at least 0.03 mg/ml. In some aspects, the concentration is at least 0.1 mg/ml. In some aspects, the concentration is at least 0.3 mg/ml.

In some aspects described herein, a concentration of polymer in the solution is less than 1 mg/ml. In some aspects, the concentration is at least 0.01 mg/ml. In some aspects, the concentration is at least 0.03 mg/ml. In some aspects, the concentration is at least 0.1 mg/ml. In some aspects, the concentration is at least 0.3 mg/ml.

In some aspects described herein, a concentration of polymer in the solution is less than 0.5 mg/ml. In some aspects, the concentration is at least 0.01 mg/ml. In some aspects, the concentration is at least 0.03 mg/ml. In some aspects, the concentration is at least 0.1 mg/ml. In some aspects, the concentration is at least 0.3 mg/ml.

In some aspects described herein, a viscosity of the solution is no more than 1000 cP (centipoise). In some aspects, the viscosity is no more than 500 cP. In some aspects, the viscosity is no more than 200 cP. In some aspects, the viscosity is no more than 100 cP. In some aspects, the viscosity is no more than 50 cP. In some aspects, the viscosity is no more than 20 cP. In some aspects, the viscosity is no more than 10 cP. In some aspects, the viscosity is no more than 5 cP. In some aspects, the viscosity is no more than 3 cP. In some aspects, the viscosity is no more than 2 cP.

Herein, viscosities of a solution are determined at a temperature of 20 °C and at a shear rate of 1 second⁻¹ (unless indicated otherwise).

In some aspects described herein, the carrier is an ophthalmically acceptable carrier. In some such aspects, the solution can be allowed to remain on the contact lens following rinsing and/or immersing in the solution, as the residual solution will not harm the eye when the contact lens is placed on the eye.

Herein, the phrase "ophthalmically acceptable carrier" refers to a carrier or a diluent that does not cause significant irritation to a subject when contacted with an eye (e.g., cornea and/or sclera) of the subject, and does not abrogate the activity and properties of the liposomes (and polymer, if present) in the solution (e.g., their ability to inhibit biofilm formation on a contact lens surface).

In some aspects described herein, the carrier is not an ophthalmically acceptable carrier. Examples of such carriers include, without limitation, carriers comprising a preservative and/or a concentration of preservative which is not ophthalmically acceptable. Such carriers may be suitable, for example, for immersing a contact lens for an extended period of time, and/or for storage for an extended period of time, while limiting the risk of bacterial growth in the solution. Typically, a solution comprising such a carrier is rinsed with an ophthalmically acceptable liquid (e.g., water, saline) solution prior to placing the contact lens on the eye.

In some aspects described herein, the solution comprises a buffer, (e.g., borate and/or phosphate), for example, such that the solution has a pH of from about 6.5 to 7.6).

In some aspects described herein, the solution is formulated as a solution suitable for storage of contact lenses (e.g., hydrogel contact lenses), for example, as known in the art.

In some aspects described herein, a contact lens is packaged in a packaging material (e.g., a blister pack) and immersed in the solution contained within a packaging material. In some aspects, the contact lens is a hydrogel contact lens, and the solution is a solution suitable for storage of hydrogel contact lenses, for example, as known in the art.

Without being bound by any particular theory, it is believed that packaging a contact lens immersed in the solution comprising liposomes (optionally with a polymer, as described herein) provides ample time for the liposome lipids (and optionally also the polymer) to coat the contact lens surface, as described herein in any one of the respective aspects, and avoids the need for a user of the contact lens to contact the contact lens with the solution.

The hydrogel may comprise any material known in the art for use in contact lens hydrogels. Examples of such hydrogel materials include, without limitation, alphafilcon (e.g., alphafilcon A), asmofilcon (e.g., asmofilcon A), balafilcon (e.g., balafilcon A), bufilcon (e.g., bufilcon A), comfilcon (e.g., comfilcon A), crofilcon, deltafilcon (e.g., deltafilcon A), dimefilcon, droxifilcon (e.g., droxifilcon A), enfilcon (e.g., enfilcon A), etafilcon (e.g., etafilcon A), filcon II, galyfilcon (e.g., galyfilcon A), hefilcon (e.g., hefilcon A, hefilcon B), hilafilcon (e.g., hilafilcon A, hilafilcon B), hioxifilcon (e.g., hioxifilcon A, hioxifilcon D), isofilcon, lidofilcon (e.g., lidofilcon A, lidofilcon B), lotrafilcon (e.g., lotrafilcon B), mafilcon, methafilcon (e.g., methafilcon A, methafilcon B), narafilcon (e.g., narafilcon A, narafilcon B), nelfilcon (e.g., nelfilcon A), ocufilcon (e.g., ocufilcon A, ocufilcon B), ofilcon (e.g., ofilcon A), omafilcon (e.g., omafilcon A), perfilcon, phemfilcon (e.g., phemfilcon A), polymacon, scafilcon (e.g., scafilcon A), senofilcon (e.g., senofilcon A), surfilcon, tefilcon, tetrafilcon (e.g., tetrafilcon A, tetrafilcon B), vifilcon (e.g., vifilcon A), and xylofilcon (e.g., xylofilcon A).

In some aspects described herein, the hydrogel comprises a polymer selected from the group consisting of poly(2-hydroxyethyl methacrylate) (e.g., cross-linked poly(2-hydroxyethyl methacrylate), polyvinyl alcohol (e.g., cross-linked polyvinyl alcohol) and a silicone. In some aspects, the polymer comprises a silicone. Such polymers may optionally comprise small amounts of additional monomers (e.g., cross-linking monomers) copolymerized with the 2-hydroxyethyl methacrylate, polyvinyl alcohol or silicone monomer. For example, 2-hydroxyethyl methacrylate may optionally be copolymerized with vinylpyrrolidone, methyl methacrylate, methacrylic acid (an anionic monomer), ethylene glycol dimethacrylate (a cross-linking monomer) and/or 3-(ethyldimethyl-ammonium)propyl methacrylamide (a cationic monomer) in a contact lens hydrogel.

In some aspects described herein, the hydrogel of the contact lens consists of a polymer which forms a hydrogel (e.g., a cross-linked polymer) and an aqueous liquid (optionally water).

As used herein the term "about" refers to ± 10 %.

The terms "comprises", "comprising", "includes", "including", "having" and their conjugates mean "including but not limited to".

The term "consisting of' means "including and limited to".

The term "consisting of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a polymer" or "at least one polymer" may include a plurality of polymers, including mixtures thereof.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, inhibiting, slowing or reversing the progression of a condition, ameliorating clinical or aesthetical symptoms of a condition or preventing the appearance of clinical or aesthetical symptoms of a condition.

### EXAMPLES

### MATERIALS AND METHODS

### Materials:

Alginic acid (from brown algae; sodium salt; medium viscosity) was obtained from Sigma-Aldrich.

Dimyristoyl phosphatidylcholine (DMPC) was obtained from Lipoid GmbH.

Gelatin (type A from porcine skin) was obtained from Sigma-Aldrich.

Hyaluronic acid (from rooster comb; sodium salt) was obtained from Sigma-Aldrich.

Hydroxypropyl methyl cellulose (HPMC; 2,600-5,600 cP at a concentration of 2 % in water at 20 °C) was obtained from Sigma.

Polyethylene oxide (PEO; average molecular weight of 200 kDa) was obtained from Sigma-Aldrich.

Polyethylene oxide dimethacrylate (PEOdMA; molecular weight 550 Da) was obtained from Sigma-Aldrich.

Poly(2-hydroxyethyl methacrylate) (polyHEMA; average molecular weight of 20 kDa) was obtained from Sigma-Aldrich.

Polyvinylpyrrolidone (PVP; average molecular weight of 40 kDa) was obtained from Sigma-Aldrich. Distilled water was purified in a Barnsted NanoPure system to 18.2 MΩ cm resistance with total organic content levels of less than approximately 1 part per billion.

All other compounds were obtained from Sigma-Aldrich.

### Hydrogel preparation:

Neat HEMA (2-hydroxyethyl methacrylate) hydrogels, containing 2 % (by molar ratio of monomers) EGDMA (ethylene glycol dimethacrylate) cross-linker (unless a different cross-linker concentration is indicated), were prepared as follows: HEMA (3.2 grams), EGDMA (100 mg for preparing a concentration of 2 %) and APS (ammonium persulfate) aqueous solution (2 ml, 53 mM) were vigorously stirred for 30 minutes until fully mixed. 50 µl TMEDA (N,N,N',N'-tetramethylethylenediamine) was added to the mixture, stirred for 20 seconds and poured into a 6 cm diameter petri dish. The gels were allowed to cross-link over 4-5 hours, followed by rinsing in distilled water for 3 days to remove unreacted materials. The obtained gels were cut into pieces for protein adsorption tests.

Liposome-loaded HEMA hydrogels, polymer-loaded hydrogels and liposome/polymer-loaded hydrogels were prepared by replacing the APS aqueous solution with a liposome or a liposome/polymer suspension with the same APS content.

MAA-PEO (methacrylamide-polyethylene oxide) hydrogels with an initial concentration of 31 weight percents methacrylamide and 15.7 weight percents PEOdMA (polyethylene oxide dimethacrylate) were prepared by photo-initiated free radical cross-linking in the presence of the water-soluble photoinitiator Irga 2959 (0.3 weight percent). The hydrogel solutions were stirred until the solution turned clear, and then they were poured into a 6 cm-diameter glass petri dish. The samples were cured by exposure to UV light for 15 minutes. For preparation of hydrogels containing liposomes, pure water was replaced by a DMPC (dimyristoyl phosphatidylcholine) multilamellar vesicle (MLV) solution with a concentration of 39 mM.

Gelatin methacrylate (GM) was synthesized according to procedures as previously described [van den Bulcke et al., Biomacromolecules 2000, 1:31-38; Hoch et al., J Mat Chem B 2013, 1:5675-5685]. Gelatin (4 grams) was dissolved in phosphate buffer (40 ml, pH 7.4) at 40 °C. The pH of the solution was then adjusted to 7.5 using an NaOH solution. 2 ml of methacrylic anhydride was added drop-wise after the gelatin was completely dissolved. During the methacrylation reaction the mixture was stirred and the pH of the solution was kept within the range of 7.0 - 7.5 for two hours of reaction time at 50 °C. Then, the mixture was diluted with PBS and dialyzed for 2 days against distilled water at 40 ° C. The reaction product was freeze-dried and stored at -20° C until use.

### Contact lenses:

The following commercial hydrogel soft contact lenses were used, and their composition, water content and modulus are summarized below. Narafilcon A (Johnson & Johnson, 1 Day TruEye®), nelfilcon A (Ciba Vision, Focus® Dailies®) and filcon II (Sauflon UK) contact lenses were originally immersed in saline solution in their aspurchased blister pack.

| **Component** | **Composition (monomers)** | **Water content** | **Lens modulus** |
|---|---|---|---|
| Narafilcon A | Silicone | 46 % | 0.66 MPa |
| Methafilcon | HEMA, MA | 55 % | |
| Nelfilcon A | Vinyl alcohol | 69% | |
| Filcon II | Silicone | 56% | 0.5 MPa |

| | | | |
|---|---|---|---|
| *HEMA*= *2-hydroxylethyl methacrylate; MA* = *methacrylic acid* | | | |

### Liposome and liposome/polymer mixture preparation:

Multilamellar vesicles (MLV) composed of dimyristoyl phosphatidylcholine (DMPC; 1,2-dimyristoyl-sn-glycero-3-phosphocholine) were prepared by hydrating the lipids at least 5 °C above the lipid melting point (T_{M}), followed by sonication in PBS (phosphate buffer saline).

Small unilamellar vesicles (SUV) of approximately 70 nm in diameter were formed by downsizing MLVs by stepwise extrusion through polycarbonate membranes starting with a 400-nm and ending with 50-nm-pore-size membrane, using a Lipex 10 ml extruder system (Northern Lipids, Vancouver, Canada).

When liposomes were mixed with polymer, the polymer solution was prepared in advance in PBS, and after full dissolution of the polymer, the solution was warmed to at least 5 °C above the lipid T_{M}, and added to the lipids, followed by mixing by stirring.

### Protein adsorption measurements:

Nonspecific protein adsorption to the hydrogels was evaluated using horseradish peroxidase (HRP)-conjugated anti-IgG adsorption, using tissue culture polystyrene (TCPS) and contact lenses soaked in PBS as controls. The samples were incubated with 1 µg/ml HRP-conjugated anti-IgG for 2 hours in a 24-well plate at 37 °C and a shaking velocity of 200 rotations per minute, followed by five rinses with phosphate-buffered saline (PBS) buffer. The samples were then transferred to new wells. 1 ml of 1 mg/ml o-phenylenediamine (OPD) in 0.1 M citrate-phosphate buffer (pH 5.0) containing 0.03 % hydrogen peroxide was added. Enzyme activity was stopped by adding an equal volume of 4 M H₂SO₄ after 15 minutes. The resulting tangerine color was measured at a wavelength of 492 nm in a 96-well plate.

### COMPARATIVE EXAMPLE 1

### Protein adsorption by hydrogels loaded with liposomes and/or polymers

Hydrogels were prepared from HEMA (2-hydroxyethyl methacrylate), MAA-PEO (methacrylamide-polyethylene oxide) or gelatin methacrylate, with or without DMPC or HSPC liposomes, as described in the Materials and Methods section hereinabove. Protein adsorption on the hydrogels was measured using anti-IgG antibodies as the protein, as described hereinabove, with results being normalized relative to adsorption on tissue culture polystyrene (TCPS) surfaces.

In order to evaluate the effect of various polymers on protein adsorption, HEMA hydrogels were prepared loaded with hyaluronic acid (HA), alginate, gelatin, hydroxypropyl methyl cellulose (HPMC), polyvinylpyrrolidone (PVP), polyethylene oxide (PEO) or non-cross-linked poly(2-hydroxyethyl methacrylate) (polyHEMA), with or without DMPC liposomes. It is to be appreciated that the loaded non-cross-linked polyHEMA differs from the polyHEMA cross-linked with EGDMA (ethylene glycol dimethacrylate) from which the HEMA hydrogel is formed.

As shown in FIG. 1, protein adsorption on a HEMA hydrogel with DMPC liposomes was about 4-fold lower than protein adsorption on a neat HEMA hydrogel (without liposomes), and more than 10-fold lower than protein adsorption on a tissue culture polystyrene surface.

As further shown therein, incorporation of the polymers alginate, HA, HPMC, PVP or PEO in a HEMA hydrogel with DMPC liposomes also resulted in reduced protein adsorption, with incorporation of HPMC, PVP or PEO further reducing protein adsorption by about 50 % as compared to HEMA with DMPC liposomes alone.

As further shown in FIG. 1, HEMA hydrogels loaded with HSPC liposomes and HPMC reduced protein adsorption in comparison with both unloaded HEMA hydrogel and HEMA hydrogel loaded with HMPC without liposomes. This result indicates that HSPC liposomes, which were in a solid phase, can reduce protein adsorption, although the reduction in protein adsorption may be to a lesser degree than that obtained with DMPC liposomes, which were in a liquid phase.

In order to evaluate the effect of hydrogel cross-linking on protein adsorption, HEMA hydrogels were prepared with 1 %, 2% or 4 % EGDMA cross-linker and loaded with DMPC MLVs and/or non-cross-linked polyHEMA.

As shown in FIG. 2, DMPC liposomes loaded into HEMA hydrogels (alone or along with non-cross-linked polyHEMA) considerable reduced protein adhesion to hydrogels with 1 %, 2% or 4% cross-linker. These results indicate that the effect of liposomes on adhesion to hydrogels occurs at a variety of degrees of hydrogel cross-linking.

In order to evaluate the effect of liposome type on protein adsorption, HEMA hydrogels loaded with DMPC multilamellar vesicles (MLVs) were compared with HEMA hydrogels loaded with DMPC small unilamellar vesicles (SUVs).

As shown in FIG. 3, DMPC MLVs and DMPC SUVs both considerably reduced protein adhesion to HEMA hydrogels. These results indicate that different types of liposomes are effective at reducing adhesion.

As shown in FIG. 4, protein adsorption on a MAA-PEO hydrogel with DMPC liposomes was lower by about 50 % than protein adsorption on a neat MAA-PEO hydrogel (without liposomes), and about 6-fold lower than protein adsorption on a tissue culture polystyrene surface.

As shown in FIG. 5, protein adsorption on a gelatin methacrylate hydrogel with DMPC liposomes was lower by about 50 % than protein adsorption on a neat gelatin methacrylate hydrogel (without liposomes), and about 8-fold lower than protein adsorption on a tissue culture polystyrene surface.

These results indicate that incorporation of liposomes in hydrogels reduces protein adsorption on the hydrogels, and that this effect of liposomes may be synergistically enhanced by incorporation of polymers such as HPMC, PVP and PEO.

The above results further indicate that the abovementioned effects occur on a variety of hydrogel types, including different species of polymer and different degrees of hydrogel cross-linking.

It is believed that the reduction in protein adsorption by liposomes is associated with a hydration layer formed on the gel surface by exposed phosphocholine groups of the liposomes.

In order to assess the resilience of the inhibitory effect on adsorption, HEMA hydrogels with various degrees of cross-linking, with and without loaded liposomes and/or polymers, were subjected to complete drying, followed by rehydration in PBS. Protein adsorption on the rehydrated hydrogels was then evaluated as described hereinabove.

As shown in FIG. 6, protein adsorption on a dried and rehydrated HEMA hydrogel loaded with DMPC liposomes (with or without alginate, HA, HPMC, PVP, PEO or gelatin) or with HSPC liposomes and HPMC was about 4-fold lower than protein adsorption on a rehydrated neat HEMA hydrogel (without liposomes) and about 10-fold lower than protein adsorption on a tissue culture polystyrene surface. These results are similar to those obtained without drying and rehydration of the hydrogel (see FIG. 1).

As shown in FIG. 7, protein adsorption on a dried and rehydrated HEMA hydrogel with 1 %, 2% or 4% cross-linker, loaded with DMPC liposomes (with or without non-cross-linked polyHEMA), was considerably lower than protein adsorption on a rehydrated neat HEMA hydrogel (without liposomes).

As shown in FIG. 8, DMPC MLVs and DMPC SUVs both considerably reduced protein adhesion to dried and rehydrated HEMA hydrogels.

These results indicate that considerable inhibition of adsorption can be obtained even after dehydration and rehydration of liposome-loaded hydrogels.

### EXAMPLE 2

### Protein adsorption by contact lenses treated with solution of liposomes and polymer

Only HA+DMPC of figure 9 and HPMC + DMPC of figure 10 fall under the scope of protection.

Soft contact lenses made from various materials (narafilcon A, nelfilcon, filcon II and methafilcon A) were removed from saline solution (in their original containers) and placed for 72 hours in a solution of a polymer (hyaluronic acid or hydroxypropyl methyl cellulose) and/or dimyristoyl phosphatidylcholine (DMPC) liposomes, or in a PBS (phosphate buffer saline) control solution. Protein adsorption on the contact lenses was measured using anti-IgG antibodies as the protein, as described in the Materials and Methods section hereinabove.

In one experiment, contact lenses made from narafilcon A, a silicone hydrogel, were immersed in a solution of 0.1 mg/ml hyaluronic acid (HA) and DMPC liposomes in the form of multilamellar vesicles (MLVs), at a DMPC concentration of 2.5 mM.

As shown in FIG. 9, protein adsorption on narafilcon A contact lenses immersed in hyaluronic acid and DMPC liposomes was reduced by 40 % relative to contact lenses immersed in PBS, whereas immersion in a solutions of DMPC liposomes alone reduced protein adsorption by 28 %, and immersion in a solution of hyaluronic acid reduced protein adsorption by 13 %.

In another experiment, nelfilcon, filcon II and methafilcon A contact lenses were immersed in a solution of 0.2 mg/ml hydroxypropyl methyl cellulose (HPMC) and DMPC liposomes in the form of small unilamellar vesicles (SUVs), at a DMPC concentration of 10 mM.

As shown in FIG. 10, protein adsorption on nelfilcon and filcon II contact lenses immersed in HPMC and DMPC liposomes was reduced by about 30 % relative to contact lenses immersed in PBS, and protein adsorption on methafilcon A contact lenses immersed in HPMC and DMPC liposomes was reduced by about 60 % relative to contact lenses immersed in PBS.

These results indicate that contact of a surface, such as a contact lens surface, with a polymer and liposomes can reduce protein adsorption on the surface.

## Claims

1. A method of inhibiting adsorption of a biofouling-promoting agent on a surface of a substrate, said substrate being composed of a hydrogel, wherein the method does not comprise a method of treatment of a human or animal body, the method comprising contacting the substrate with a composition which comprises liposomes and a polymer, wherein said liposomes comprise at least one phospholipid, said at least one phospholipid comprising at least one phosphatidylcholine, and a concentration of phospholipids in said liposomes in said composition is in a range of from 1 mM to 150 mM, wherein said liposomes are **characterized by** a melting point below a temperature of said surface during said contacting, and wherein said polymer is selected from the group consisting of hyaluronic acid and hydroxypropyl methyl cellulose.

2. A method of inhibiting biofilm formation on a surface of a substrate, said substrate being composed of a hydrogel, wherein the method does not comprise a method of treatment of a human or animal body, the method comprising contacting the substrate with a composition which comprises liposomes and a polymer, wherein said liposomes comprise at least one phospholipid, said at least one phospholipid comprising at least one phosphatidylcholine, and a concentration of phospholipids in said liposomes in said composition is in a range of from 1 mM to 150 mM, wherein said liposomes are **characterized by** a melting point below a temperature of said surface during said contacting, and wherein said polymer is selected from the group consisting of hyaluronic acid and hydroxypropyl methyl cellulose.

3. The method of claim 1 or claim 2, wherein said contacting comprises incorporating said composition which comprises liposomes within said hydrogel.

4. The method of any one of claims 1 to 3, wherein said substrate is prepared by forming said hydrogel in the presence of said liposomes.

5. The method of any one of claims 1 and 2, wherein said composition further comprises a hydrogel.

6. The method of claim 1, wherein said biofouling-promoting agent is selected from the group consisting of a biofouling-promoting protein and a biofouling-promoting polysaccharide.

7. The method of claim 2, wherein said substrate is a contact lens, the method comprising rinsing and/or immersing the contact lens in a solution comprising said liposomes, said polymer, and an aqueous carrier.

## Patentansprüche

1. Verfahren zur Hemmung der Adsorption eines biofäulnisfördernden Mittels auf einer Oberfläche eines Substrats, wobei das Substrat aus einem Hydrogel besteht, wobei das Verfahren kein Verfahren zur Behandlung eines menschlichen oder tierischen Körpers umfasst, wobei das Verfahren das Inkontaktbringen des Substrats mit einer Zusammensetzung umfasst, die Liposomen und ein Polymer umfasst, wobei die Liposomen mindestens ein Phospholipid umfassen, wobei das mindestens eine Phospholipid mindestens ein Phosphatidylcholin umfasst und eine Konzentration von Phospholipiden in den Liposomen in der Zusammensetzung in einem Bereich von 1 mM bis 150 mM liegt, wobei die Liposomen durch einen Schmelzpunkt unterhalb einer Temperatur der Oberfläche während des Inkontaktbringens gekennzeichnet sind, und wobei das Polymer ausgewählt ist aus der Gruppe, bestehend aus Hyaluronsäure und Hydroxypropylmethylcellulose.

2. Verfahren zur Hemmung von Biofilmbildung auf einer Oberfläche eines Substrats, wobei das Substrat aus einem Hydrogel besteht, wobei das Verfahren kein Verfahren zur Behandlung eines menschlichen oder tierischen Körpers umfasst, wobei das Verfahren das Inkontaktbringen des Substrats mit einer Zusammensetzung umfasst, die Liposomen und ein Polymer umfasst, wobei die Liposomen mindestens ein Phospholipid umfassen, wobei das mindestens eine Phospholipid mindestens ein Phosphatidylcholin umfasst und eine Konzentration von Phospholipiden in den Liposomen in der Zusammensetzung in einem Bereich von 1 mM bis 150 mM liegt, wobei die Liposomen durch einen Schmelzpunkt unterhalb einer Temperatur der Oberfläche während des Inkontaktbringens gekennzeichnet sind, und wobei das Polymer ausgewählt ist aus der Gruppe, bestehend aus Hyaluronsäure und Hydroxypropylmethylcellulose.

3. Verfahren nach Anspruch 1 oder Anspruch 2, wobei das Inkontaktbringen das Einbringen der Zusammensetzung, die Liposomen umfasst, in das Hydrogel umfasst.

4. Verfahren nach einem der Ansprüche 1 bis 3, wobei das Substrat durch Bilden des Hydrogels in Gegenwart der Liposomen hergestellt wird.

5. Verfahren nach einem der Ansprüche 1 und 2, wobei die Zusammensetzung ferner ein Hydrogel umfasst.

6. Verfahren nach Anspruch 1, wobei das biofäulnisfördernde Mittel ausgewählt ist aus der Gruppe, bestehend aus einem biofäulnisfördernden Protein und einem biofäulnisfördernden Polysaccharid.

7. Verfahren nach Anspruch 2, wobei das Substrat eine Kontaktlinse ist, wobei das Verfahren das Spülen und/oder Eintauchen der Kontaktlinse in eine Lösung umfasst, die die Liposomen, das Polymer und einen wässrigen Träger umfasst.

## Revendications

1. Procédé d'inhibition de l'adsorption d'un agent favorisant l'encrassement biologique sur une surface d'un substrat, ledit substrat étant composé d'un hydrogel, le procédé ne comprenant pas de procédé de traitement d'un corps humain ou animal et le procédé comprenant la mise en contact du substrat avec une composition qui comprend des liposomes et un polymère, lesdits liposomes comprenant au moins un phospholipide, ledit au moins un phospholipide comprenant au moins une phosphatidylcholine et une concentration de phospholipides dans lesdits liposomes dans ladite composition étant dans une plage de 1 mM à 150 mM, lesdits liposomes étant **caractérisés par** un point de fusion inférieur à une température de ladite surface pendant ladite mise en contact et ledit polymère étant choisi dans le groupe constitué par l'acide hyaluronique et l'hydroxypropylméthylcellulose.

2. Procédé d'inhibition de la formation d'un biofilm sur une surface d'un substrat, ledit substrat étant composé d'un hydrogel, le procédé ne comprenant pas de procédé de traitement d'un corps humain ou animal et le procédé comprenant la mise en contact du substrat avec une composition qui comprend des liposomes et un polymère, lesdits liposomes comprenant au moins un phospholipide, ledit au moins un phospholipide comprenant au moins une phosphatidylcholine et une concentration de phospholipides dans lesdits liposomes dans ladite composition étant dans une plage de 1 mM à 150 mM, lesdits liposomes étant **caractérisés par** un point de fusion inférieur à une température de ladite surface pendant ladite mise en contact et ledit polymère étant choisi dans le groupe constitué par l'acide hyaluronique et l'hydroxypropylméthylcellulose.

3. Procédé selon la revendication 1 ou la revendication 2, dans lequel ladite mise en contact comprend l'incorporation de ladite composition comprenant des liposomes dans ledit hydrogel.

4. Procédé selon l'une quelconque des revendications 1 à 3, dans lequel ledit substrat est préparé par formation dudit hydrogel en présence desdits liposomes.

5. Procédé selon l'une quelconque des revendications 1 et 2, dans lequel ladite composition comprend en outre un hydrogel.

6. Procédé selon la revendication 1, dans lequel ledit agent favorisant l'encrassement biologique est choisi dans le groupe constitué d'une protéine favorisant l'encrassement biologique et d'un polysaccharide favorisant l'encrassement biologique.

7. Procédé selon la revendication 2, dans lequel ledit substrat est une lentille de contact, le procédé comprenant le rinçage et/ou l'immersion de la lentille de contact dans une solution comprenant lesdits liposomes, ledit polymère et un support aqueux.
